# FASCICULE DE BREVET EUROPEEN

(11) **EP 0 816 352 B1**
(45) Date de publication et mention de la délivrance du brevet: **21.02.2001**
(21) Numéro de dépôt: 97401319.5
(22) Date de dépôt: 11.06.1997
(51) Int. Cl.: C07D 307/92, C07D 405/04, C07D 409/04, C07D 417/04, A61K 31/335, A61K 31/38

(54) **Nouveaux composés biaryles hétérocycliques et leur utilisation en médecine humaine ou vétérinaire ainsi qu'en cosmétique**
Biaryl heterocyclische Verbindungen und ihre Verwendung in der Human- oder Tiermedizin und in der Kosmetik
Bi-aryl heterocyclic compounds and their use in human or veterinary medicine and in cosmetics

(30) Priorité: 28.06.1996 FR 9608115
(43) Date de publication de la demande: 07.01.1998
(73) Titulaire: CENTRE INTERNATIONAL DE RECHERCHES DERMATOLOGIQUES GALDERMA (C.I.R.D. GALDERMA), F-06560 Valbonne (FR)
(72) Inventeur: Diaz, Philippe, Les Jardins de Saint-Antoine, 06200 Nice (FR); Thoreau, Etienne, 06140 Vence (FR)
(74) Mandataire: Tezier Herman, Béatrice

(56) Documents cités:
- EP-A- 0 070 531
- EP-A- 0 292 348
- GB-A- 2 164 648
- CHEMICAL ABSTRACTS, vol. 118, no. 15, 12 avril 1993 Columbus, Ohio, US; abstract no. 147416, JIANG J. ET AL.: "Reaction of aromatic epoxides with beta-naphtol catalyzed by titanium chloride" XP002025187 & WUJI HUAXUE XUEBAO, vol. 8, no. 2, 1992, CH., pages 206-208,

## Description

La présente invention a pour objet, de nouveaux composés biaryl hétérocycliques aromatiques, leur procédé de préparation et leur utilisation en médecine humaine ou vétérinaire ainsi qu'en cosmétique.

Le document EP A- 0 292 348 décrit des composés rétinoides de type hétérocycliques polycycliques. Ces composés présentent une partie hétérocyclique insaturée et la substitution par le cycle aromatique est en position 2 sur la partie hétérocyclique.

Les composés selon l'invention présentent une activité dans les domaines de la différenciation et de la prolifération cellulaire. En conséquence, ces composés peuvent être utilisés dans le traitement topique et systémique des affections dermatologiques liées à un désordre de la kératinisation, des affections dermatologiques (ou autres) à composante inflammatoire, virale et/ou immunoallergique et des proliférations dermiques ou épidermiques, qu'elles soient bénignes ou malignes. Ces composés peuvent être utilisés en outre, dans le traitement des maladies de dégénérescence du tissu conjonctif, pour lutter contre le vieillissement de la peau, qu'il soit photoinduit ou chronologique et traiter les troubles de la cicatrisation. Ils trouvent enfin une application dans le domaine ophtalmologique, notamment dans le traitement des cornéopathies.

On peut également les utiliser dans les compositions cosmétiques pour l'hygiène corporelle ou capillaire.

Les composés selon l'invention sont représentés par la formule générale (I) suivante: 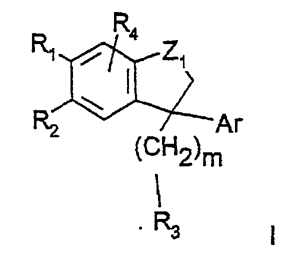 dans laquelle
Z₁ représente O, S, N-r',
R₁ et R₂ pris ensemble forment avec le cycle aromatique adjacent un cycle à 5 ou 6 chainons éventuellement substitué par un ou plusieurs groupes méthyle et/ou éventuellement interrompu par un radical SO, un radical SO₂, un atome d'oxygène ou de soufre,
R₃ représente
   (i) un atome d'hydrogène, un radical alkyle inférieur, un radical alkényle inférieur, un radical alkynyle inférieur, un atome d'halogène, un radical cyano ou un radical -O-R₇, R₇ ayant la signification donnée ci-après,
   (ii) un radical 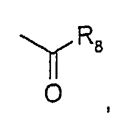 R₈ ayant la signification donnée ci-après,
   (iii) un radical 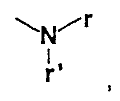 r et r' ayant la signification donnée ci-après,
R4 représente:
   (i) un atome d'hydrogène,
   (ii) un radical alkyle inférieur,
   (iii) un atome d'halogène,
   (iv) un radical -OR₇, R₇ ayant la signification donnée ci-après,
   (v) un radical acyle inférieur,
Ar représente un radical choisi parmi les radicaux de formules (a)-(h) suivantes: 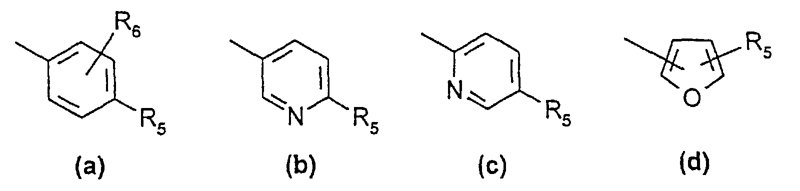 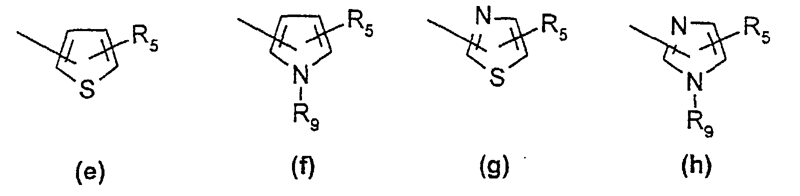 dans lesquelles
   R₅ représente :
   (i) le radical -CH₃,
   (ii) le radical -(CH₂)ₚ-O-R₇,
   (iii) un radical 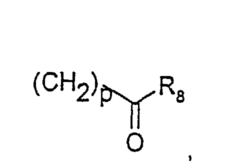
   (iv) un radical 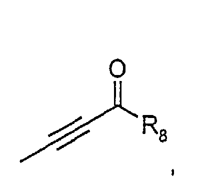
   (v) un radical 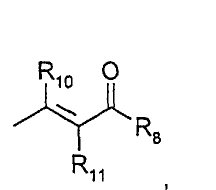
   R₇, R₈, R₉, R₁₀, R₁₁ et p ayant les significations données ci-après,
R₆ représente un atome d'hydrogène, un atome d'halogène, un radical alkyle inférieur, un radical acyle inférieur ou le radical -OR₇, R₇ ayant la signification donnée ci-après,
R₇ identique ou différent, représente un atome d'hydrogène, un radical alkyle inférieur, un radical aryle, un radical aralkyle, un radical polyhydroxyalkyle ou un radical polyéther, un radical acyle inférieur,
R₈, identique ou différent, représente :
   (a) un atome d'hydrogène, un radical alkyle inférieur,
   (b) un radical 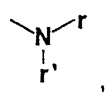 r et r' , ayant les significations données ci-après
   (c) un radical -OR₁₂
R₉ représente un atome d'hydrogène, un radical alkyle inférieur ou un radical acyle inférieur,
R₁₀ et R₁₁, identiques ou différents, représentent un atome d'hydrogène ou un radical alkyle inférieur
R₁₂ représente un atome d'hydrogène, un radical alkyle inférieur, un radical mono ou polyhydroxyalkyle, un radical aryle ou aralkyle éventuellement substitué(s) ou un reste de sucre ou d'aminoacide,
r et r', identiques ou différents, représentent des groupements protecteurs de fonctions amines, un atome d'hydrogène, un radical alkyle inférieur, un reste d'amino acide ou de sucre, ou encore pris ensemble, forment un hétérocycle,
m représente 0 ou 1,
p, identique ou différent, représente 0, 1, 2 ou 3,
ainsi que leurs sels, leurs racémiques, leurs isomères optiques purs ou leurs mélanges en toutes proportions.

Lorsque les composés selon l'invention se présentent sous forme de sels pharmaceutiquement ou cosmétiquement acceptables obtenus par addition d'une base; il s'agit notamment de sels d'un métal alcalin ou alcalino-terreux ou encore de zinc ou d'une amine organique.

Lorsque les composés se présentent sous forme de sels, par addition d'un acide; il s'agit notamment de sels pharmaceutiquement ou cosmétiquement acceptables obtenus par addition d'un acide minéral ou organique, en particulier l'acide chlorhydrique, sulfurique, acétique, citrique, fumarique, hémisuccinique, maléique et mandélique.

Par radical alkyle inférieur on entend un radical ayant de 1 à 6 atomes de carbone linéaire ou ramifié éventuellement substitué par un ou plusieurs atomes d'halogène et de préférence les radicaux méthyle, éthyle, isopropyle, butyle, tertiobutyle et hexyle.

Par radical alkényle inférieur on entend un radical ayant de 1 à 6 atomes de carbone linéaire ou ramifié comportant une ou plusieurs doubles liaisons et de préférence les radicaux allyle ou vinyle.

Par radical alkynyle inférieur on entend un radical ayant de 1 à 6 atomes de carbone linéaire ou ramifié comportant une à plusieurs triple liaisons

Par radical acyle inférieur, on entend un radical ayant de 1 à 6 atomes de carbone et de préférence, les radicaux acétyle, propionyle ou pivaloyle,

Par groupement protecteur de fonction amine, on entend les groupements correspondants décrits dans "Protecting groups in organic synthesis" by T.W Greene, Ed. by John Wiley and Sons (1981).

Par radical cycloalkyle, on entend un radical alcane cyclique ou polycyclique contenant de 1 à 10 atomes de carbone, éventuellement substitué par un ou des atomes d'halogène ou un ou des radicaux hydroxyle et de préférence les radicaux adamantyle ou 1-méthylcyclohéxyle.

Par radical polyéther, on entend un radical ayant de 1 à 6 atomes de carbone et de 1 à 3 atomes d' oxygène ou de soufre tel que les radicaux méthoxyméthyl éther, méthoxyéthoxyméthyl éther ou méthylthyométhyl éther.

Par radical polyhydroxyalkyle, on doit entendre un radical contenant de 1 à 6 atomes de carbone et de 1 à 5 groupes hydroxyles tels que les radicaux 2,3-dihydroxypropyle, 2,3,4-trihydroxybutyle, 2,3,4,5-tétrahydroxypentyle ou le reste du pentaérythritol.

Par radical aryle éventuellement substitué, on doit entendre un radical phényle, éventuellement substitué par un ou plusieurs atomes d'halogène, une fonction hydroxyle ou fonction nitro, un groupe méthoxy ou une fonction amine éventuellement substituée.

Par radical aralkyle éventuellement substitué, on doit entendre le radical benzyle ou phénéthyle éventuellement substitué par un ou plusieurs atomes d'halogène, une fonction hydroxyle ou nitro, ou un groupe méthoxy.

Par reste d'aminoacide on doit entendre un reste dérivant par exemple de l'un des 20 aminoacides de configuration L ou D constitutifs de protéines de mammifères.

Par reste de sucre, on doit entendre un reste dérivant par exemple du glucose, du galactose, du mannose ou de l'acide glucuronique.

Par hétérocycle, on entend de préférence un radical pipéridino, morpholino, pyrrolidino ou pipérazino, éventuellement substitué en position 4 par un radical alkyle en C₁-C₆ ou mono ou polyhydroxyalkyle tels que définis ci-dessus.

Selon la présente invention, les composés de formule (I) plus particulièrement préférés sont ceux pour lesquels l'une au moins, et de préférence toutes, les conditions ci-dessous sont respectées:
- R₃ est un hydrogène,
- R₄ est un hydrogène,
- R₅ est un radical de formule (iii), (iv) ou (v),
- R₆ est un hydrogène,
- R₈ est un radical OR₁₂,
- R₁₂ est un hydrogène ou un radical alkyle inférieur,
- Ar est un radical de formule (a),
- Z₁ est un atome d'oxygène,
- m=1,
- p=0.

Parmi les composés entrant dans le cadre de la présente invention, on peut notamment citer les exemples suivants:
- Acide 4-(3,5,5,8,8-pentaméthyl-2,3,5,6,7,8-hexahydronaphto[2,3-b]furan-3-yl)-benzoïque
- Acide (+) 4-(3,5,5,8,8-pentaméthyl-2,3,5,6,7,8-hexahydronaphto[2,3-b]furan-3-yl)-benzoïque
- Acide 4-(3,5,5,8,8-pentaméthyl-2,3,5,6,7,8-hexahydronaphto[2,3-b]thiophene-3-yl)-benzoïque
- Acide (-) 4-(3,5,5,8,8-pentaméthyl-2,3,5,6,7,8-hexahydronaphto[2,3-b]furan-3-yl)-benzoïque
- 4-(3,5,5,8,8-pentaméthyl-2,3,5,6,7,8-hexahydronaphto[2,3-b]furan-3-yl)-benzoate d'éthyle
- (+) 4-(3,5,5,8,8-pentaméthyl-2,3,5,6,7,8-hexahydronaphto[2,3-b]furan-3-yl)-benzoate d'éthyle
- (-) 4-(3,5,5,8,8-pentaméthyl-2,3,5,6,7,8-hexahydronaphto[2,3-b]furan-3-yl)-benzoate d'éthyle
- [5-(3,5,5,8,8-pentamethyl-2,3,5,6,7,8-hexahydronaphto[2,3-b]furan-3-yl)-thiophene-3-yl] carboxylate de methyle
- acide [5-(3,5,5,8,8-pentamethyl-2,3,5,6,7,8-hexahydronaphto[2,3-b]furan-3-yl)-thiophene-3-yl] carboxylique.
- acide [5-(3,5,5,8,8-pentamethyl-2,3,5,6,7,8-hexahydronaphto[2,3-b]furan-3-yl)-thiophen-2-yl]-carboxylique.
- [5-(3,5,5,8,8-pentamethyl-2,3,5,6,7,8-hexahydronaphto[2,3-b]furan-3-yl)-thiophen-3-yl]-methanol
- [5-(3,5,5,8,8-pentamethyl-2,3,5,6,7,8-hexahydronaphto[2,3-b]furan-3-yl)-thiophen-3-yl]-carbaldehyde
- [5-(3,5,5,8,8-pentamethyl-2,3,5,6,7,8-hexahydronaphto[2,3-b]furan-3-yl)-thiophene-3-yl]-acrylate d'éthyle.
- acide [5-(3,5,5,8,8-pentamethyl-2,3,5,6,7,8-hexahydronaphto[2,3-b]furan-3-yl)-thiophene-3-yl] acrylique.
- [5-(3,5,5,8,8-pentamethyl-2,3,5,6,7,8-hexahydronaphto[2,3-b]furan-3-yl)-thiophene-3-yl]-propynoate de methyl.
- acide [5-(3,5,5, 8,8-pentamethyl-2,3,5,6,7,8-hexahydronaphto[2,3-b]furan-3-yl)-thiophene-3-yl]-propynoique.

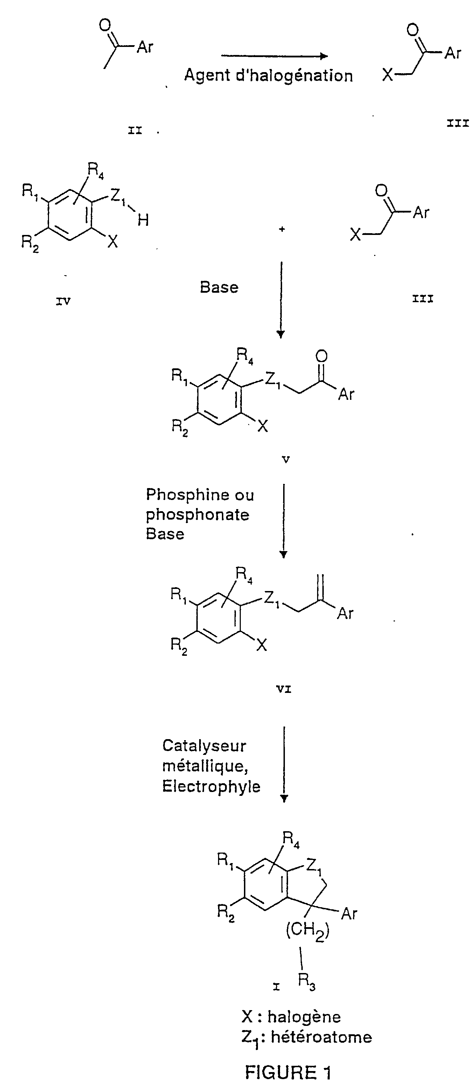

La présente invention a également pour objet les procédés de préparation des composés de formule (I), en particulier selon les schémas réactionnels donnés à la figure 1.

Ainsi les composés de formule générale (I) peuvent être obtenus (figure 1) à partir de la cétone Il, par halogénation, par exemple au moyen d'un agent de bromation tel que le brome. Le composé III obtenu est ensuite couplé avec le composé IV, en présence de base telle que le carbonate de potassium ou l' hydrure de sodium. Le dérivé couplé V est soumis à l'action d'une phosphine ou d'un phosphonate en présence d'une base pour conduire au composé VI. Le composé VI est cyclisé par action d'un catalyseur métallique comme le diacétate de palladium, en présence d'un donneur d'hydrure comme l'acide formique ou d'un nucléophile comme le vinyl tributyl étain ou l'acétate de lithium et si nécessaire d'une base. L'adjonction de sels ou de zéolites d'argent comme Ag₃PO₄ et de phosphines chirales comme le Binap permet d'induire une cylisation asymetrique.

Les produits de formule générale (I) peuvent servir de produits de départ pour la fabrication d'autres composés. Ces dérivés seront obtenus selon les méthodes classiques de synthèse employées en chimie, telles que celles décrites dans "Advanced Organic Chemistry" de J. March; John Willey and Sons, 1985.

Par exemple, on peut procéder aux modifications fonctionnelles du groupe R 5 comme indiqué ci-dessous:
acide carboxylique → ester
ester → acide carboxylique
acide → chlorure d' acide
chlorure d'acide → amide
acide → amide
acide → alcool
alcool → aldéhyde
amide →amine
thiol →thioéther
thioéther → sulfoxyde
thioéther → sulfone
acide sulfonique → ester sulfonique
acide sulfonique → sulfonamide
acide sulfinique → ester sulfinique
La présente invention a également pour objet à titre de médicament les composés de formule (I) tels que décrits ci-dessus.

Certains de ces composés présentent une activité dans un test qui consiste à identifier des molécules agonistes des RXRs, tel que décrit dans la demande de brevet français n°95-07301 déposé le 19 juin 1995 par la Demanderesse. Ce test comprend les étapes suivante: (i) on applique topiquement sur une partie de la peau d'un mammifère une quantité suffisante d'un composé qui est un ligand actif d'au moins un récepteur de la superfamille des récepteurs stéroïdiens/thyroïdiens, autre qu'un ligand spécifique des récepteurs RXRs, et pouvant s'hétérodimériser avec les RXRs, tel q'une molécule agoniste des RARs, (ii) on administre par voie systémique ou topique sur cette même partie de la peau du mammifère, avant, pendant ou après l'étape (i), une molécule susceptible de présenter une activité agoniste des RXRs et (iii) on évalue la réponse sur la partie de la peau ainsi traitée du mammifère. Ainsi, la réponse à une application topique sur l'oreille d'un mammifère d'une molécule agoniste des RARs qui correspond à une augmentation de l'épaisseur de cette oreille peut être augmentée par l'administration par voie systémique ou topique d'une molécule agoniste des RXRs. Certains des composés selon l'invention présentent également une activité dans le test de différenciation des cellules (F9) de tératocarcinome embryonnaire de souris (Cancer Research 43, p. 5268, 1983) et/ou dans le test d'inhibition de l'ornithine décarboxylase après induction par le TPA chez la souris (Cancer research 38, p. 793-801, 1978). Ces tests montrent les activités de ces composés respectivement dans les domaines de la différenciation et de la prolifération cellulaire.

Les composés selon l'invention conviennent particulièrement bien dans les traitement des affections dermatologiques, rhumatismales, respiratoires ainsi qu'ophtalmologiques, en particulier dans les domaines de traitement suivants :
1) Pour traiter les affections dermatologiques liées à un désordre de la kératinisation portant sur la différenciation et sur la prolifération notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle.
2) Pour traiter d'autres types de trouble de la kératinisation, notamment les ichtyoses, les états ichtyosiformes, la maladie de Darier, les kératodermies palmoplantaires, les leucoplasies et les états leucoplasiformes, le lichen cutané ou muqueux (buccal).
3) Pour traiter d'autres affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriatique, ou encore l'atopie cutanée, telle que l'eczéma ou l'atopie respiratoire ou encore l'hypertrophie gingivale; les composés peuvent également être utilisés dans certaines affections inflammatoires ne présentant pas de trouble de la kératinisation.
4) Pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides et les proliférations pouvant être induites par les ultra-violets notamment dans le cas des épithélioma baso et spinocellulaires.
5)Pour traiter d'autres désordres dermatologiques tels que les dermatoses bulleuses et les maladies du collagène.
6) Pour traiter certains troubles ophtalmologiques, notamment les cornéopathies.
7) Pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique.
8) Pour prévenir ou guérir les stigmates de l'atrophie épidermique et/ou dermique induite par les corticostéroïdes locaux ou systémiques, ou tout autre forme d'atrophie cutanée.
9) Pour prévenir ou traiter les troubles de la cicatrisation ou pour prévenir ou réparer les vergetures.
10) Pour lutter contre les troubles de la fonction sébacée tels que l'hyperséborrhée de l'acné ou la séborrhée simple.
11) Dans le traitement ou la prévention des états cancéreux ou précancéreux, plus particulièrement les leucémies promyélocytaires.
12) Dans le traitement d'affections inflammatoires telles que l'arthrite.
13) Dans le traitement de toute affection d'origine virale au niveau cutané, telle que le syndrome de Kaposis, ou général.
14) Dans la prévention ou le traitement de l'alopécie.
15) Dans le traitement d'affections dermatologiques ou générales à composante immunologique.
16) Dans le traitement d'affections du système cardiovasculaire telles que l'artériosclérose ou l'hypertension ainsi que le diabète non-insulino dépendant.
17) dans le traitement de désordres cutanés dus à une exposition aux rayonnements U.V..

Dans les domaines thérapeutiques mentionnés ci-dessus, les composés selon l'invention peuvent être avantageusement employés en combinaison avec d'autres composés à activité de type rétinoïdes, avec des dérivés de la vitamine D, avec des corticostéroïdes ou des estrogènes, en association avec des antioxydants, avec des α-hydroxy ou α-céto acides ou leurs dérivés, ou encore avec des bloqueurs de canaux potassiques.

Par vitamines D ou leurs dérivés on entend par exemple les dérivés de la vitamine D₂ ou D₃ et en particulier la 1,25-dihydroxyvitamine D₃.

Par antiradicaux libres on entend par exemple l'α-tocophérol, la Super Oxyde Dismutase, l'Ubiquinol ou certains chélatants de métaux.

Par α-hydroxy ou α-céto acides ou leurs dérivés, on entend par exemple les acides lactique, malique, citrique, glycolique, mandélique, tartrique, glycérique,ascorbique ou dérivés d'acide salicylique ou leurs sels, amides ou esters.

Par bloqueurs de canaux potassiques, on entend par exemple le Minoxidil (2,4-diamino-6-pipéridino-pyrimidine-3-oxyde) et ses dérivés.

La présente invention a donc aussi pour objet une nouvelle composition pharmaceutique destinée notamment au traitement des affections susmentionées, caractérisée par le fait qu'elle comporte, dans un support pharmaceutiquement acceptable au moins un composé de formule I telle que définie ci-dessus.

L'administration des composés selon l'invention peut être effectuée par voie entérale, parentérale, topique ou oculaire.

Par voie entérale, les médicaments peuvent se présenter sous forme de comprimés, de gélules, de dragées, de sirops, de suspensions, de solutions, de poudres, de granulés, d'émulsions, de microsphères ou nanosphères ou vésicules lipidiques ou polymériques permettant une libération contrôlée. Par voie parentérale, les compositions peuvent se présenter sous forme de solutions ou suspensions pour perfusion ou pour injection. Les composés selon l'invention sont généralement administrés à une dose journalière d'environ 0,01 mg/kg à 100 mg/kg en poids corporel en 1 à 3 prises.

Par voie topique, les compositions pharmaceutiques à base de composés selon l'invention sont destinées au traitement de la peau et des muqueuses et se présentent sous forme d'onguents, de crèmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques et d'hydrogels permettant une libération contrôlée. Ces compositions par voie topique peuvent se présenter soit sous forme anhydre, soit sous forme aqueuse selon l'indication clinique.

Par voie occulaire, ce sont principalement des collyres.

Ces compositions pharmaceutiques contiennent au moins un composé de formule I telle que définie ci-dessus, à une concentration de préférence comprise entre 0,001 et 5 % en poids par rapport au poids total de la composition.

Les composés de formule (I) selon l'invention, trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les effets néfastes du soleil ou dans le traitement des peaux physiologiquement sèches, pour prévenir et/ou lutter contre le vieillissement photo-induit ou chronologique.

Dans le domaine cosmétique, les composés selon l'invention peuvent être avantageusement employés en combinaison avec d'autres composés à activité de type rétinoïdes, avec les vitamines D ou leurs dérivés, avec des corticostéroïdes, en association avec des anti-radicaux libres, avec des α-hydroxy ou α-céto acides ou leurs dérivés, ou encore avec des bloqueurs de canaux ioniques.

Les différents produits pris en association avec les composés de la présente invention étant tels que définis ci-dessus.

La présente invention concerne donc également une composition cosmétique contenant, dans un support cosmétiquement acceptable, au moins un composé de formule telle que définie ci-dessus. Cette composition se présente notamment sous forme d'une crème, d'un lait, d'une lotion, d'un gel, de microsphères ou nanosphères ou vésicules lipidiques ou polymériques, d'un savon ou d'un shampooing.

La concentration en composé de formule (I) dans les compositions cosmétiques est comprise avantageusement entre 0,001 et 3 % en poids par rapport au poids total de la composition.

Les compositions médicamenteuses et cosmétiques selon l'invention peuvent, en outre, contenir des additifs inertes ou même pharmacodynamiquement ou cosmétiquement actifs ou des combinaisons de ces additifs et notamment : des agents mouillants; des agents dépigmentants tels que l'hydroquinone, l'acide azelaïque, l'acide caféïque ou l'acide kojique; des émollients; des agents hydratants comme le glycérol, le PEG 400, la thiamorpholinone et ses dérivés ou l'urée; des agents antiséborrhéiques ou antiacnéiques, tels que la S-carboxyméthylcystéine, la S-benzyl-cystéamine, leurs sels et leurs dérivés, ou le péroxyde de benzoyle; des antibiotiques comme l'érythromycine et ses esters, la néomycine, la clindamycine et ses esters, les tétracyclines; des agents antifongiques tels que le kétoconazole ou les polyméthylène-4,5 isothiazolinones-3 ; des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4- benzothiadiazine 1,1-dioxyde) et le Phénytoïn (5,4-diphényl-imidazolidine 2,4-dione) ; des agents anti-inflammatoires non stéroïdiens; des caroténoïdes et, notamment le β-carotène; des agents antipsoriatiques tels que l'anthraline et ses dérivés et enfin, les acides eicosa-5,8,11,14-tétraynoïque et eicosa-5,8,11-trynoïque, leurs esters et les amides.

Les compositions selon l'invention peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'α-tocophérol, le butylhydroxyanisole ou le butylhydroxytoluène.

On va maintenant donner, à titre d'illustration et sans aucun caractère limitatif, plusieurs exemples de préparation des composés actifs de formule I selon l'invention ainsi que des exemples de compositions les contenant.

### A. EXEMPLES DE COMPOSES SELON LA VOIE DE SYNTHESE ILLUSTREE PAR LA FIGURE 1

### EXEMPLE 1

### 4-(3,5,5,8,8-PENTAMETHYL-2,3,5,6,7,8-HEXAHYDRONAPHTO[2,3-B]FURAN-3-YL)-BENZOATE D'ETHYLE

### 3-Iodo-5,5,8,8-tétramethyl-5,6,7,8-tétrahydronaphtalen-2-ol

On additionne goutte à goutte une solution de perchlorate de sodium à 3,6% à un mélange de 5,5,8,8-tétramethyl-5,6,7,8-tétrahydronaphtalen-2-ol ( 6,35 g, 31,1 mmol), de soude (1,23 g, 34,2 mmol), d'iodure de sodium (4,6 g, 31,1 mmol) dans le méthanol (115 ml), à 0°C. On laisse agiter deux heures à 0°C. On additionne 33 ml d'une solution de thiosulfate de sodium à 10%. Après agitation, on acidifie par de l'acide chlorhydrique jusqu'à pH 1. On extrait par 200 ml d'éther éthylique. La phase organique est lavée deux fois par 400 ml d'eau, séchée sur sulfate de magnésium et concentrée à l'évaporateur rotatif sous vide à 40°C.
Le produit est recristallisé dans l'heptane.

Solide blanc. Masse: 5,67 g. Rendement: 55%. Pf: 103°C
RMN ¹H (CDCl₃, 250 MHz): 1,23 (6H, s), 1,24 (6H, s), 1,64 (4H, s), 5,04 (1H, s), 6,93 (1H Ar, s), 7,59 (1H Ar, s).
RMN ¹³C (CDCL₃, 250 MHz):31,49, 31,75, 33,53, 34,19, 34,63, 34,76, 82,86, 112,47, 135,93, 139,73, 147,67, 152,16.

### 4-Bromoacéty benzoate d'éthyle.

Une solution de brome (1,6 ml) dans 15 ml de CH₂Cl₂ est additionnée goutte à goutte à une solution de 4-acéthyl benzoate d'éthyle (5,28 g, 27 mmol) de dioxane (30 ml) et d'éther éthylique (30 ml). L'agitation est poursuivie 1h puis le mélange est versé dans 100 g de glace et extrait par 100 ml d'éther éthylique. La phase organique est lavée deux fois par 100 ml d'eau, séchée sur sulfate de magnésium et concentrée à l'évaporateur rotatif sous vide.
Le produit est purifié par recristallisation dans l'heptane

Solide blanc. Masse: 5,03 g, Rendement: 69%. Pf: 71°C
RMN ¹H (CDCL₃, 250 MHz): 1,42 (3H, t, J=7,5Hz), 4,42 (2H, q, J=7,5Hz), 4,48 (2H, s), 8,04 (2H Ar, d, J=8,75 Hz), 8,16 (2H Ar, d, J=8,75 Hz).
RMN ¹³C (CDCL₃, 250 MHz):14,18, 30,64, 61,50, 128,74, 129,87, 134,90, 136,97, 165,37, 190,76.

### 4-[(3-iodo-5,5,8,8-tétraméthyl-5,6,7,8-tétrahydro-naphtalen-2-yloxy)-acétyl]-benzoate d'éthyle.

Une solution de 3-lodo-5,5,8,8-tétramethyl-5,6,7,8-tétrahydronaphtalen-2-ol (5,6 g, 17 mmol), de 4-Bromoacéty benzoate d'éthyle (4,6 g, 17 mmol),et de carbonate de potassium (2,4 g, 17,4 mmol) dans la méthyléthylcétone (150 ml), est chauffée à reflux pendant 8h. On filtre puis on concentre à l'évaporateur rotatif, le milieu réactionnel. On ajoute 200 ml d'eau et 200 ml d'éther éthylique. Après agitation et décantation, la phase organique est lavée deux fois par 200 ml d' eau, séchée sur sulfate de magnésium et concentrée à l'évaporateur rotatif sous vide à 40 °C. Le produit est purifié par chromatographie flash sur colonne de silice (CH₂Cl₂ 80 %, heptane 20 %).

Solide blanc. Masse: 4,7 g. Rendement: 53%. Pf. 137°C.
RMN ¹H (CDCL₃, 250 MHz): 1,20 (6H, s), 1,22 (6H, s), 1,42 (3H, t, J=7,5Hz), 1,63 (4H, s), 4,41 (2H, q, J=7,5Hz), 5,21 (2H, s), 6,68 (1H Ar, s), 7,64 (1H Ar, s), 8,11 (2H Ar, d, J=8,75 Hz), 8,14 (2H Ar, d, J=8,75 Hz).
RMN ¹³C (CDCL₃, 250 MHz): 14,05, 31,41, 31,53, 33,53, 34,55, 34,60, 61,30, 72,58, 83,27, 110,75, 128,41, 129,60, 134,62, 137,44, 137,65, 140,88, 146,68, 154,12, 165,40, 194,86.

### 4-[(3-iodo-5,5,8,8-tétraméthyl-5,6,7,8-tétrahydro-naphtalen-2-yloxy)-1-propényl]-benzoate d'éthyle.

On additionne en 8 heures, à un mélange de 4-[(3-iodo-5,5,8,8-tétraméthyl-5,6,7,8-tétrahydro naphtalen-2-yloxy)-acétyl]-benzoate d'éthyle (3,6 g, 7 mmol) et de bromure de méthyltryphénylphoshine (3,4 g, 9,5 mmol) dans le THF (40 ml), une solution de méthylate de sodium à 30 % (1,31 ml).
On agite 36 h à température ambiante. Le mélange est concentré à l'évaporateur rotatif sous vide à 40°C.On extrait par 90 ml d'éther éthylique et 90 ml d'eau. Après décantation la phase organique est lavée deux fois par 90 ml d'eau, séchée sur sulfate de magnésium anhydre et concentrée à l'évaporateur rotatif sous vide à 40°C. Le produit est purifié par chromatographie flash sur colonne de silice (CH₂Cl₂ 60 %, heptane 40 %).

Solide blanc. Masse: 2,79 g, Rendement: 77%. Pf: 88°C
RMN ¹H (CDCL₃, 250 MHz): 1,24 (6H, s), 1,25 (6H, s), 1,40 (3H, t, J=7,5Hz), 1,65 (4H, s), 4,39 (2H, q, J=7,5Hz), 4,91 (2H, s), 5,71 (2H, s), 6,76 (1H Ar, s), 7,57 (2H Ar, d, J=8,75 Hz), 7,65 (1H Ar, s), 8,04 (2H Ar, d, J=8,75 Hz).
RMN ¹³C (CDCL₃, 250 MHz): 14,33, 31,71, 31,80, 33,72, 34,56, 34,90, 60,95, 70,60, 83,93, 110,70, 116,98, 126,18, 126,24, 129,71, 129,75, 129,88, 137,58, 140,25, 142,26, 142,77, 146,61, 154,70, 166,33.

### 4-(3,5,5,8,8-pentaméthyl-2,3,5,6,7,8-hexahydronaphto[2,3-b]furan-3-yl)-benzoate d'éthyle

On chauffe à 60°C pendant 4 h un mélange de tributhylamine (0,55 ml, 3,8 mmol), de diacétate de palladium (22 mg, 0,1 mmol), d'acide formique (0,042 ml, 1,1 mmol) et de 4-[(3-iodo-5,5,8,8-tétraméthyl-5,6,7,8-tétrahydro naphtalen-2-yloxy)-1-propényl]-benzoate d'éthyle (520 mg, 1 mmol) dans l'acétonitrile (15 ml). Le milieu réactionnel est concentré à l'évaporateur rotatif sous vide à 40°C. On ajoute 20 ml d'eau et 20 ml d'éther d'éthylique. Après décantation, la phase organique est lavée deux fois par 20 ml d'eau, séchée sur sulfate de magnésium, et concentrée à l'évaporateur rotatif sous vide à;40°C.
Le produit est purifié par chromatographie flash sur colonne de silice (CH₂Cl₂ 80% heptane 20 %)
Solide blanc. Masse: 315 mg, Rendement: 80%. Pf: 139°C
RMN ¹H (CDCL₃, 250 MHz): 1,18 (3H, s), 1,22 (3H, s), 1,28 (6H, s), 1,38 (3H, t, J=7,5Hz), 1,66 (4H, s), 1,75 (3H, s), 4,36 (2H, q, J=7,5Hz), 4,42 (2H, d, J=8,75Hz), 4,52 (2H, d, J=8,75Hz), 6,81 (1H Ar, s), 6,94 (1H Ar, s), 7,36 (2H Ar, d, J=8,75 Hz), 7,98 (2H Ar, d, J=8,75 Hz).
RMN ¹³C (CDCL₃, 250 MHz): 14,34, 26,07, 32,01, 32,09, 32,28, 34,15, 34,72, 35,14, 35,24, 50,27, 60,87, 85,69, 107,07, 121,74, 126,48, 128,69, 129,66, 132,20, 137,78, 145,61, 151,84, 157,63, 166,43.

### EXEMPLE 2

### ACIDE 4-(3,5,5,8,8-PENTAMETHYL-2,3,5,6,7,8-HEXAHYDRONAPHTO[2,3-B] FURAN-3-YL)-BENZOÏQUE

Un mélange de 4-(3,5,5,8,8-pentaméthyl-2,3,5,6,7,8-hexahydronaphto[2,3-b] furan-3-yl)-benzoate d'éthyle (200 mg, 0,5 mmol), de soude (100 mg, 2,4 mmol), et d'hydroxyde de lithium (100 mg, 2,4 mmol) dans 5 ml d'une solution de THF, méthanol et d'eau (5/1/1) est chauffé à reflux 24h. Après concentration à l'évaporateur rotatif sous vide à 40°C. Après rajout de 5 ml d'eau et 5 ml d'éther éthylique et acidification par une solution d'acide chlorhydrique concentrée jusqu'à pH 1, la phase organique est lavée deux fois par 5 ml d'eau, séchée sur sulfate de magnésium, et concentrée à l'évaporateur rotatif sous vide à 40°C. Le solide obtenu est lavé à l'heptane.
Solide blanc. Masse: 152 mg, Rendement: 82%. Pf: 245°C
RMN ¹H (DMSO, 250 MHz): 1,19 (3H, s), 1,23 (3H, s), 1,29 (6H, s), 1,66 (4H, s), 1,77 (3H, s), 4,44 (2H, q, J=8,5Hz), 4,54 (2H, d, J=8,5Hz), 6,82 (1H Ar, s), 6,95 (1H Ar, s), 7,40 (2H Ar, d, J=8,75 Hz), 8,05 (2H Ar, d, J=8,75 Hz).
RMN ¹³C (DMSO, 250 MHz): 26,43, 32,43, 32,51, 32,70, 34,58, 35,15, 35,54, 35,64, 50,77, 86,06, 107,55, 122,16, 127,08, 127,85, 130,78, 132,48, 138,28, 146,13, 153,45, 158,04, 172,23.

### EXEMPLE 3

### (+)-4-(3,5,5,8,8-PENTAMETHYL-2,3,5,6,7,8-HEXAHYDRONAPHTO[2,3-B] FURAN-3-YL)-BENZOATE D'ETHYLE

On chauffe à 60°C pendant 4j un mélange de carbonate de calcium (200 mg, 2 mmol), de diacétate de palladium (22 mg, 0,1 mmol), formiate de sodium (136 mg, 2 mmol) et de 4-[(3-iodo-5,5,8,8-tétraméthyl-5,6,7,8-tétrahydro naphtalen-2-yloxy)-1-propényl]-benzoate d'éthyle (520 mg, 1 mmol), (S)-(-)-2,2'-Bis(diphénylphosphino)-1,1'-binaphtyl et de zeolite d'argent (Aldrich 36,660-9) dans l'acétonitrile (15 ml). Le milieu réactionnel est filtré sur célite, concentré à l'évaporateur rotatif sous vide à 40°C. On ajoute 20 ml d'eau et 20 ml d'éther d'éthylique. Après décantation, la phase organique est lavée deux fois par 20 ml d'eau, séchée sur sulfate de magnésium, et concentrée à l'évaporateur rotatif sous vide à;40°C.
Le produit est purifié par chromatographie flash sur colonne de silice (CH₂Cl₂ 80% heptane 20 %)
Solide blanc. Masse: 105 mg, Rendement: 27%. Pf: 139°C, α_{d}[CHCl₃]: +122,4.
RMN ¹H (CDCL₃, 250 MHz): 1,18 (3H, s), 1,22 (3H, s), 1,28 (6H, s), 1,38 (3H, t, J=7,5Hz), 1,66 (4H, s), 1,75 (3H, s), 4,36 (2H, q, J=7,5Hz), 4,42 (2H, d, J=8,75Hz), 4,52 (2H, d, J=8,75Hz), 6,81 (1H Ar, s), 6,94 (1H Ar, s), 7,36 (2H Ar, d, J=8,75 Hz), 7,98 (2H Ar, d, J=8,75 Hz).
RMN ¹³C (CDCL₃, 250 MHz): 14,34, 26,07, 32,01, 32,09, 32,28, 34,15, 34,72, 35,14, 35,24, 50,27, 60,87, 85,69, 107,07, 121,74, 126,48, 128,69, 129,66, 132,20, 137,78, 145,61, 151,84, 157,63, 166,43.

### EXEMPLE 4

### ACIDE (+)-4-(3,5,5,8,8-PENTAMETHYL-2,3,5,6,7,8-HEXAHYDRONAPHTO[2,3-B]FURAN-3-YL)-BENZOÏQUE

Un mélange de (+)-4-(3,5,5,8,8-pentaméthyl-2,3,5,6,7,8-hexahydronaphto[2,3-b] furan-3-yl)-benzoate d'éthyle (200 mg, 0,5 mmol), de soude (100 mg, 2,4 mmol), et d'hydroxyde de lithium (100 mg, 2,4 mmol) dans 5 ml d'une solution de THF, méthanol et d'eau (5/1/1) est chauffé à reflux 24h. Après concentration à l'évaporateur rotatif sous vide à 40°C. Après rajout de 5 ml d'eau et 5 ml d'éther éthylique et acidification par une solution d'acide chlorhydrique concentrée jusqu'à pH 1, la phase organique est lavée deux fois par 5 ml d'eau, séchée sur sulfate de magnésium, et concentrée à l'évaporateur rotatif sous vide à 40°C. Le solide obtenu est lavé à l'heptane.
Solide blanc. Masse: 152 mg, Rendement: 82%. Pf: 245°C
RMN ¹H (DMSO, 250 MHz): 1,19 (3H, s), 1,23 (3H, s), 1,29 (6H, s), 1,66 (4H, s), 1,77 (3H, s), 4,44 (2H, q, J=8,5Hz), 4,54 (2H, d, J=8,5Hz), 6,82 (1H Ar, s), 6,95 (1H Ar, s), 7,40 (2H Ar, d, J=8,75 Hz), 8,05 (2H Ar, d, J=8,75 Hz).
RMN ¹³C (DMSO, 250 MHz): 26,43, 32,43, 32,51, 32,70, 34,58, 35,15, 35,54, 35,64, 50,77, 86,06, 107,55, 122,16, 127,08, 127,85, 130,78, 132,48, 138,28, 146,13, 153,45, 158,04, 172,23.

### EXEMPLE 5

### (-)-4-(3,5,5,8,8-PENTAMETHYL-2,3,5,6,7,8-HEXAHYDRONAPHTO[2,3-B] FURAN-3-YL)-BENZOATE D'ETHYLE

On chauffe à 60°C pendant 4j un mélange de carbonate de calcium (200 mg, 2 mmol), de diacétate de palladium (22 mg, 0,1 mmol), formiate de sodium (136 mg, 2 mmol) et de 4-[(3-iodo-5,5,8,8-tétraméthyl-5,6,7,8-tétrahydro naphtalen-2-yloxy)-1-propényl]-benzoate d'éthyle (520 mg, 1 mmol), (R)-(+)-2,2'-Bis(diphénylphosphino)-1,1'-binaphtyl et de zeolite d'argent (Aldrich 36,660-9) dans l'acétonitrile (15 ml). Le milieu réactionnel est filtré sur célite, concentré à l'évaporateur rotatif sous vide à 40°C. On ajoute 20 ml d'eau et 20 ml d'éther d'éthylique. Après décantation, la phase organique est lavée deux fois par 20 ml d'eau, séchée sur sulfate de magnésium, et concentrée à l'évaporateur rotatif sous vide à;40°C.
Le produit est purifié par chromatographie flash sur colonne de silice (CH₂Cl₂ 80% heptane 20 %)
Solide blanc. Masse: 85 mg, Rendement: 22%. Pf: 139°C, α_{d}[CHCl₃]: -110,2.
RMN ¹H (CDCl₃, 250 MHz): 1,18 (3H, s), 1,22 (3H, s), 1,28 (6H, s), 1,38 (3H, t, J=7,5Hz), 1,66 (4H, s), 1,75 (3H, s), 4,36 (2H, q, J=7,5Hz), 4,42 (2H, d, J=8,75Hz), 4,52 (2H, d, J=8,75Hz), 6,81 (1H Ar, s), 6,94 (1H Ar, s), 7,36 (2H Ar, d, J=8,75 Hz), 7,98 (2H Ar, d, J=8,75 Hz).
RMN ¹³C (CDCl₃, 250 MHz): 14,34, 26,07, 32,01, 32,09, 32,28, 34,15, 34,72, 35,14, 35,24, 50,27, 60,87, 85,69, 107,07, 121,74, 126,48, 128,69, 129,66, 132,20, 137,78, 145,61, 151,84, 157,63, 166,43.

### EXEMPLE 6

### ACIDE (-)-4-(3,5,5,8,8-PENTAMETHYL-2,3,5,6,7,8-HEXAHYDRONAPHTO[2,3-B]FURAN-3-YL)-BENZOÏQUE

Un mélange de (-)-4-(3,5,5,8,8-pentaméthyl-2,3,5,6,7,8-hexahydronaphto[2,3-b] furan-3-yl)-benzoate d'éthyle (200 mg, 0,5 mmol), de soude (100 mg, 2,4 mmol), et d'hydroxyde de lithium (100 mg, 2,4 mmol) dans 5 ml d'une solution de THF, méthanol et d'eau (5/1/1) est chauffé à reflux 24h. Après concentration à l'évaporateur rotatif sous vide à 40°C. Après rajout de 5 ml d'eau et 5 ml d'éther éthylique et acidification par une solution d'acide chlorhydrique concentrée jusqu'à pH 1, la phase organique est lavée deux fois par 5 ml d'eau, séchée sur sulfate de magnésium, et concentrée à l'évaporateur rotatif sous vide à 40°C. Le solide obtenu est lavé à l'heptane.
Solide blanc. Masse: 152 mg, Rendement: 82%. Pf: 245°C
RMN ¹H (DMSO, 250 MHz): 1,19 (3H, s), 1,23 (3H, s), 1,29 (6H, s), 1,66 (4H, s), 1,77 (3H, s), 4,44 (2H, q, J=8,5Hz), 4,54 (2H, d, J=8,5Hz), 6,82 (1H Ar, s), 6,95 (1H Ar, s), 7,40 (2H Ar, d, J=8,75 Hz), 8,05 (2H Ar, d, J=8,75 Hz).
RMN ¹³C (DMSO, 250 MHz): 26,43, 32,43, 32,51, 32,70, 34,58, 35,15, 35,54, 35,64, 50,77, 86,06, 107,55, 122,16, 127,08, 127,85, 130,78, 132,48, 138,28, 146,13, 153,45, 158,04, 172,23.

### EXEMPLE 7

### [5-(3,5,5,8,8-PENTAMETHYL-2,3,5,6,7,8-HEXAHYDRONAPHTO[2,3-B] FURAN-3-YL)-THIOPHENE-3-YL] CARBOXYLATE DE METHYLE

### 5-Bromoacétyl-3-thiophène carboxylate de méthyle.

Une solution de bromure de bromoacétyle (13,52 g, 0,067 mol) dans 20 ml de CH₂Cl₂ est additionnée goutte à goutte à une solution de chlorure d'aluminium (13,4g, 0,105 mol) dans 50 ml de CH₂Cl₂ à 0°C. Le mélange est agité 1h à 0°C, puis une solution de 3-thiophène carboxylate de méthyle (9,49 g, 0,067 mol) dans le CH₂Cl₂ (50 ml) est additionnée goutte à goutte. L'agitation est poursuivie 12h puis le mélange est versé sur de l'eau et de la glace et extrait au CH₂Cl₂. La phase organique est lavée deux fois à l'eau, séchée sur sulfate de magnésium et concentrée à l'évaporateur rotatif sous vide.
Le produit est purifié par chromatographie flash sur colonne de silice (AcOEt 20 %, heptane 80 %).
Solide blanc. Masse: 5,95 g, Rendement: 34%. F= 103-104°C
RMN ¹H (CDCl₃, 250 MHz): 3,91 (3H, s), 4,36 (2Hs), 8,17 (1H Ar), 8,40 (1H Ar).

### 5-[(3-iodo-5,5,8,8-tétraméthyl-5,6,7,8-tétrahydronaphtalen-2-yloxy)-acétyl]-3-thiophène carboxylate de méthyle.

Une solution de 3-lodo-5,5,8,8-tétramethyl-5,6,7,8-tétrahydronaphtalen-2-ol (4g, 12,1 mmol), de 5-Bromoacétyl-3-thiophène carboxylate de méthyle (3,18g, 12,1mmol),et de carbonate de potassium (1,84 g, 13,3mmol) dans la méthyléthylcétone (100 ml), est chauffée à reflux pendant 4h. On filtre puis on concentre à l'évaporateur rotatif, le milieu réactionnel. Le mélange est extrait à l'éther éthylique. La phase organique est lavée à l'eau, séchée sur sulfate de magnésium et concentrée à l'évaporateur rotatif sous vide à 40 °C. Le produit est purifié par chromatographie flash sur colonne de silice (CH₂Cl₂ 60 %, heptane 40 %).
Solide blanc. Masse: 6,2 g. Rendement: 68%. F=97°C.
RMN ¹H (CDCl₃, 250 MHz): 1,21 (6H, s), 1,23 (6H, s), 1,63 (4H, s), 3,89 (3H, s), 5,05 (2H, s), 6,71 (1H Ar, s), 7,66 (1H Ar, s), 8,39 (1H Ar, s), 8,57 (1H Ars).

### 5-[1-(3-iodo-5,5,8,8-tétraméthyl-5,6,7,8-tétrahydronaphtalen-2-yloxyméthyl)-vinyl]-3-thiophène carboxylate de méthyle.

On additionne en 8 heures, à un mélange de 5-[(3-iodo-5,5,8,8-tétraméthyl-5,6,7,8-tétrahydronaphtalen-2-yloxy)-acétyl]-3-thiophène carboxylate de méthyle (4,1 g, 8mmol) et de bromure de méthyltriphénylphoshine (3,8g, 10,8mmol) dans le THF (50 ml), une solution de méthylate de sodium à 30 % dans le méthanol (1,68ml).
On agite 12 h à température ambiante. Le mélange est concentré à l'évaporateur rotatif sous vide à 40°C.On extrait à l'éther éthylique et lave à d'eau. La phase organique est ensuite séchée sur sulfate de magnésium et concentrée à l'évaporateur rotatif sous vide à 40°C. Le produit est purifié par chromatographie flash sur colonne de silice (CH₂Cl₂ 60 %, heptane 40 %).
Solide blanc. Masse: 3,51 g, Rendement: 86%. F= 94°C
RMN ¹H (CDCl₃, 250 MHz): 1,23 (6H, s), 1,25 (6H, s), 1,64 (4H, s), 3,85 (3H, s), 4,86 (2H, s), 5,59 (1H, s), 5,67 (1H, s), 6,77 (1H Ar, s), 7,58 (1H Ar, s), 7,66 (1H Ar, s), 7,96 (1H Ar, s).
RMN ¹³C (CDCl₃, 250 MHz): 32,28, 32,19, 34,18, 35,03, 35,36, 52,29, 70,54, 84,37, 115,22, 111,17, 124,76, 132,14, 138,08, 134,12, 136,65, 140,81, 142,76, 147,11, 155,03, 163,44.

### [5-(3,5,5,8,8-pentaméthyl-2,3,5,6,7,8-hexahydronaphto[2,3-b] furan-3-yl)-thiophène-3-yl] carboxylate de méthyle.

On chauffe à 60°C pendant 4 h un mélange de tributylamine (3,64ml, 25,16mmol), de diacétate de palladium (74mg, 0,33 mmol), d'acide formique (0,28ml, 7,28mmol) et de 5-[1-(3-iodo-5,5,8,8-tétraméthyl-5,6,7,8-tétrahydronaphtalen-2-yloxyméthyl)-vinyl]-3-thiophène carboxylate de méthyle (3,38g, 6,62mmol) dans l'acétonitrile (100ml). Le milieu réactionnel est concentré à l'évaporateur rotatif sous vide à 40°C, traité par de l'eau et extrait à l'éther d'éthylique. Après décantation, la phase organique est lavée deux fois à l'eau, séchée sur sulfate de magnésium, et concentrée à l'évaporateur rotatif sous vide à 40°C.
Le produit est purifié par chromatographie flash sur colonne de silice (CH₂Cl₂ 70% heptane 30 %)
Solide blanc. Masse: 2,12 g, Rendement: 83%. F= 117°C
RMN ¹H (CDCl₃, 250 MHz): 1,20 (3H, s), 1,23 (3H, s), 1,26 (3H, s), 1,27 (3H, s), 1,65 (4H, s), 1,77 (3H, s), 3,84 (3H, s), 4,40 (1H, d, J=8,6 Hz), 4,52 (2H, d, J=8,6 Hz), 6,79 (1H Ar, s), 7,06 (1H Ar, s), 7,30 (1H Ar, d, J=1,2 Hz), 7,93 (1H Ar, d, J=1,2 Hz).
RMN ¹³C (CDCl₃, 250 MHz): 27,25, 31,95, 32,02, 32,08, 32,26, 34,17, 34,75, 35,11, 35,19, 48,27, 51,78, 85,42, 107,23, 121,42, 124,04, 131,50, 131,80, 133,00, 137,93, 146,13, 152,87, 157,19, 163,29.

### EXEMPLE 8

### ACIDE [5-(3,5,5,8,8-PENTAMETHYL-2,3,5,6,7,8-HEXAHYDRONAPHTO[2,3-b] FURAN-3-YL)-THIOPHENE-3-YL] CARBOXYLIQUE.

Un mélange de [5-(3,5,5,8,8-pentaméthyl-2,3,5,6,7,8-hexahydronaphto[2,3-b] furan-3-yl)-thiophene-3-yl] carboxylate de méthyle (2,1g, 5,4 mmol), d'hydroxyde de lithium (2g, 48 mmol, de méthanol (1ml) et d'eau (1ml) dans 30 ml de THF est chauffé à reflux 6h. Après concentration à l'évaporateur rotatif sous vide à 40°C, rajout d'eau, d'éther éthylique et acidification par une solution d'acide chlorhydrique concentrée jusqu'à pH 1, la phase organique est lavée deux fois à l'eau, séchée sur sulfate de magnésium, et concentrée à l'évaporateur rotatif sous vide à 40°C. Le solide obtenu est lavé à l'heptane.
Solide blanc. Masse: 1,8 g, Rendement: 92%. F= 237°C
RMN ¹H (DMSO et CDCl₃, 250 MHz): 1,20 (3H, s), 1,23 (3H, s), 1,26 (3H, s), 1,27 (3H, s), 1,65 (4H, s), 1,76 (3H, s), 4,40 (1H, d, J=8,6 Hz), 4,52 (2H, d, J=8,6 Hz), 6,77 (1H Ar, s), 7,07 (1H Ar, s), 7,29 (1H Ar, s), 7,93 (1H Ar, s).
RMN ¹³C (DMSO et CDCl₃, 250 MHz): 27,17, 31,84, 31,90, 31,98, 32,13, 34,02, 34,59, 34,97, 35,05, 48,10, 85,29, 106,99, 121,34, 124,23, 131,36, 131,78, 134,00, 137,73, 145,85, 152,39, 157,05, 164,59.

### EXEMPLE 9

### ACIDE [5-(3,5,5,8,8-PENTAMETHYL-2,3,5,6,7,8-HEXAHYDRONAPHTO[2,3-b] FURAN-3-YL)-THIOPHEN-2-YL]-CARBOXYLIQUE.

### 2-Bromoacétyl-5-bromothiophène.

Une solution de brome (2,9 ml, 56,5 mmol) dans 30 ml de CH₂Cl₂ est additionnée goutte à goutte à une solution de 2-acétyl-5-bromothiophène (10g, 49mmol) de dioxane (55 ml) et d'éther éthylique (55 ml). L'agitation est poursuivie 1h puis le milieu réactionnel est versé dans un mélange eau/glace et extrait à l'éther éthylique. La phase organique est lavée deux fois à l'eau, séchée sur sulfate de magnésium et concentrée à l'évaporateur rotatif sous vide. Le produit est purifié par recristallisation dans l'heptane
Solide blanc. Masse: 9,63 g, Rendement: 70%. F= 92°C.
RMN ¹H (CDCl₃, 250 MHz): 4,28 (2H, s), 7,14 (1H Ar, d, J=4Hz), 7,55 (1H Ar, d, J=4Hz).
RMN ¹³C (CDCl₃, 250 MHz): 29,83, 131,74, 133,92, 183,60

### 5-bromo-2-[(3-iodo-5,5,8,8-tétraméthyl-5,6,7,8-tétrahydronaphtalen-2-yloxy)acétyl] thiophène.

Une solution de 3-lodo-5,5,8,8-tétramethyl-5,6,7,8-tétrahydronaphtalen-2-ol (4g, 12,1 mmol), de 2-Bromoacétyl-5-bromothiophène (3,44g, 12,1 mmol),et de carbonate de potassium (1,8g, 13,3 mmol) dans la méthyléthylcétone (100 ml), est chauffée à reflux pendant 3h. On filtre puis on concentre à l'évaporateur rotatif, le milieu réactionnel, on ajoute de l'eau et de l'éther éthylique. Après agitation et décantation, la phase organique est lavée deux fois à l'eau, séchée sur sulfate de magnésium et concentrée à l'évaporateur rotatif sous vide à 40 °C. Le produit est purifié par chromatographie flash sur colonne de silice (CH₂Cl₂ 5 %, heptane 95 %).
Solide blanc. Masse: 4,86 g. Rendement: 75%. F= 90°C.
RMN ¹H (CDCl₃, 250 MHz): 1,20 (6H, s), 1,22 (6H, s), 1,63 (4H, s), 4,97 (2H, s), 6,69 (1H Ar, s), 7,14 (1H Ar, d, J=4Hz), 7,65 (1H Ar, s), 7,96 (1H Ar, d, J=4Hz).
RMN ¹³C (CDCl₃, 250 MHz): 31,74, 31,61, 33,76, 34,80, 73,09, 83,07, 124,32, 141,20, 147,10, 154,20, 110,70, 131,60, 134,90, 137,90, 187,70.

### 5-bromo-2-[1-(3-iodo-5,5,8,8-tétraméthyl-5,6,7,8-tétrahydronaphtalen-2-yloxyméthyl)-vinyl] thiophène.

On additionne en 8 heures, à un mélange de 5-bromo-2-[(3-iodo-5,5,8,8-tétraméthyl-5,6,7,8-tétrahydronaphtalen-2-yloxy)-acétyl] thiophène (4,3 g, 7,9 mmol) et de bromure de méthyltryphénylphoshine (3,8 g, 10,7 mmol) dans le THF (45 ml), une solution de méthylate de sodium à 30 % dans le méthanol (1,65 ml, 8,66 mmol).
On agite 12 h à température ambiante. Le mélange est concentré à l'évaporateur rotatif sous vide à 40°C.On extrait à l'éther éthylique et lave à d'eau. La phase organique est ensuite séchée sur sulfate de magnésium et concentrée à l'évaporateur rotatif sous vide à 40°C. Le produit est purifié par chromatographie flash sur colonne de silice (CH₂Cl₂ 20 %, heptane 80 %).
Solide blanc. Masse: 3,26 g, Rendement: 78%. F= 71°C
RMN ¹H (CDCl₃, 250 MHz): 1,24 (12H, s), 1,64 (4H, s), 4,81 (2H, s), 5,47 (1H, s), 5,56 (1H, s), 6,75 (1H Ar, s), 6,96 (2H Ar, s), 7,65 (1H Ar, s).
RMN ¹³C (CDCl₃, 250 MHz): 32,10, 32,20, 34,13, 34,96, 35,27, 70,76, 84,21, 111,13, 125,15, 130,74, 138,03, 112,03, 136,60, 140,78, 143,64, 147,05, 154,99.

### 5-bromo-2-(3,5,5,8,8-pentaméthyl-2,3,5,6,7,8-hexahydronaphto[2,3-b] furan-3-yl)- thiophène.

On chauffe à 60°C pendant 4 h un mélange de tributylamine (2,66 ml, 18,39 mmol), de diacétate de palladium (53 mg, 0,24 mmol), d'acide formique (0,205 ml, 5,32 mmol) et de 5-bromo-2-[1-(3-iodo-5,5,8,8-tétraméthyl-5,6,7,8-tétrahydronaphtalen-2-yloxyméthyl)-vinyl] thiophène (2,57g, 4,84mmol) dans l'acétonitrile (75 ml). Le milieu réactionnel est concentré à l'évaporateur rotatif sous vide à 40°C. On ajoute de l'eau et de l'éther éthylique. Après décantation, la phase organique est lavée deux fois par 20 ml d'eau, séchée sur sulfate de magnésium, et concentrée à l'évaporateur rotatif sous vide à;40°C.
Le produit est purifié par chromatographie flash sur colonne de silice (CH₂Cl₂ 20% heptane 80 %)
Solide blanc. Masse: 2 mg, Rendement: 44%. F= 110°C
RMN ¹H (CDCl₃, 250 MHz): 1,24 (6H, s), 1,26 (6H, s), 1,66 (4H, s), 1,72 (3H, s), 4,36 (1H, q, J=8,6Hz), 4,48 (1H, d, J=8,6Hz) 6,55 (1H Ar, d, J=3,7Hz), 6,78 (1H Ar, s), 6,86 (1H Ar, d, J=3,8Hz), 7,07 (1H Ar, s).
RMN ¹³C (CDCl₃, 250 MHz): 27,1, 31,1, 32,0, 32,1, 32,3, 34,2, 34,7, 35,1, 35,2, 48,5, 85,4, 107,2, 121,5, 124,0, 129,6, 110,5, 131,4, 137,9, 146,1, 153,4, 157,3.

### Acide [5-(3,5,5,8,8-pentaméthyl-2,3,5,6,7,8-hexahydronaphto[2,3-b] furan-3-yl)-thiophen-2-yl]-carboxylique.

Un cristal d'iode et du magnésium (58,3mg, 2,39mmol) dans 0,5 ml de THF sont chauffés puis une solution de 5-bromo-2-(3,5,5,8,8-pentaméthyl-2,3,5,6,7,8-hexahydronaphto[2,3-b]-furan-3-yl)-thiophène (770 mg, 1,9mmol) dans le THF (7ml), est additionnée goutte à goutte. Le mélange est ensuite chauffé à reflux 4h, puis refroidi à température ambiante. On fait buller du dioxide de carbone pendant 15mn, et l'agitation est poursuivie 12h. La solution est traitée par de l'éther éthylique et acidifiée par une solution d'acide chlorhydrique 2N jusqu'à pH 1, la phase organique est lavée deux fois à l'eau, séchée sur sulfate de magnésium, et concentrée à l'évaporateur rotatif sous vide à 40°C. Le solide obtenu est recristallisé dans un mélange heptane/éther éthylique.
Solide blanc. Masse: 383 mg, Rendement: 54%. F= 216°C
RMN ¹H (CDCl₃, 250 MHz): 1,22 (6H, s), 1,24 (3H, s), 1,66 (4H, s), 1,79 (3H, s), 4,42 (1H, d, J=8,6Hz), 4,54 (1H, d, J=8,6Hz), 6,80 (1H Ar, s), 6,85 (1H Ar, d, J=3,9Hz), 7,08 (1H Ar, s), 7,72 (1H Ar, d, J=3,9Hz).
RMN ¹³C (CDCl₃, 250 MHz): 27,1, 32,0, 32,1, 32,3, 34,8, 34,2, 35,1, 35,2, 48,8, 85,4, 107,4, 121,4, 124,9, 131,2, 135,2, 138,1, 146,3, 157,3, 161,6, 166,9.

### EXEMPLE 10

### [5-(3,5,5,8,8-PENTAMETHYL-2,3,5,6,7,8-HEXAHYDRONAPHTO[2,3-b] FURAN-3-YL)-THIOPHEN-3-YL]-METHANOL

Une solution 1M d'hydrure de diisobutylaluminium dans le toluène (4,2ml, 4,2mmol) est additionnée à 0°C, goutte à goutte à une solution de 5-(3,5,5,8,8-pentaméthyl-2,3,5,6,7,8-hexahydronaphto[2,3-b]-furan-3-yl)-thiophène-3-carboxylate de méthyle (737mg, 1,92mmol) dans le toluène (30ml). La soultution est agitatée 2h à 0°C, puis traité par une solution de tartrate double de sodium et potassium filtrée et reprise dans un mélange d'éther éthylique et d'eau. La phase organique est lavée à l'eau, séchée sur sulfate de magnésium et concentrée à l'évaporateur rotatif sous vide à 40°C.
Solide blanc. Masse: 720 mg, Rendement: quantitatif. F= 127°C
RMN ¹H (CDCl₃, 250 MHz): 1,22 (3H, s), 1,24 (3H, s), 1,27 (6H, s), 1,66 (4H, s), 1,75 (3H, s), 4,38 (1H, d, J=8,5Hz), 4,52 (1H, d, J=8,5Hz), 4,60 (1H, s), 6,78 (1H Ar, s), 6,82 (1H Ar, s), 7,07 (1H Ar, s), 7,10 (1H Ar, s).
RMN ¹³C (CDCl₃, 250 MHz):27,58, 31,98, 32,03, 32,11, 32,28, 34,18, 35,16, 35,25, 48,37, 61,01, 85,62, 107,09, 120,73, 121,57, 123,50, 132,06, 137,72, 141,87, 145,86, 152,98, 157,27.

### EXEMPLE 11

### [5-(3,5,5,8,8-PENTAMETHYL-2,3,5,6,7,8-HEXAHYDRONAPHTO[2,3-b] FURAN-3-YL)-THIOPHEN-3-YL]-CARBALDEHYDE

Un mélange de [5-(3,5,5,8,8-pentaméthyl-2,3,5,6,7,8-hexahydronaphto[2,3-b] furan-3-yl)-thiophen-3-yl]-méthanol (1,4g, 3,93mmol) et de dichromate de pyridinium (2,8g, 7,4mmol) dans le CH₂Cl₂ (100ml) est agité 3h à T.A., puis filtré sur silice et concentré à l'évaporateur rotatif sous vide à 40°C.
Solide blanc. Masse: 1 g, Rendement: 72%. F= 138°C
RMN ¹H (CDCl₃, 250 MHz): 1,21 (3H, s), 1,24 (3H, s), 1,27 (3H, s), 1,28 (3H, s), 1,66 (4H, s), 1,79 (3H, s), 4,41 (1H, d, J=8,6Hz), 4,53 (1H, d, J=8,6Hz), 6,80 (1H Ar, s), 7,06 (1H Ar, s), 7,31 (1H Ar, s), 7,96 (1H Ar, s) , 9,81 (1H Ar, s).
RMN ¹³C (CDCl₃, 250 MHz): 27,02, 31,96, 32,02, 32,10, 32,27, 34,19, 34,78, 35,10, 35,18, 48,35, 85,30, 107,34, 121,16, 121,38, 131,55, 136,08, 138,07, 142,79, 146,34, 154,66, 157,22, 185,19.

### EXEMPLE 12

### [5-(3,5,5,8,8-PENTAMETHYL-2,3,5,6,7,8-HEXAHYDRONAPHTO[2,3-B] FURAN-3-YL)-THIOPHENE-3-YL]-ACRYLATE D'ETHYLE.

De l'hydrure de sodium à 80% dans l'huile (44mg, 1,47mmol), est additionné à un mélange de [5-(3,5,5,8,8-pentaméthyl-2,3,5,6,7,8-hexahydronaphto[2,3-b]furan-3-yl)-thiophen-3-yl]-carbaldehyde (400mg, 1,13mmol) et de triéthylphosphonoacétate (304mg, 1,35mmol) dans le THF (10ml). Le mélange est agité 4h à température ambiante, extrait à l'éther éthylique et lavé à l'eau. Après séchage la phase organique est concentré à l'évaporateur rotatif sous vide à 40°C, le produit est purifié par chromatographie flash sur colonne de silice.
Solide blanc. Masse: 430 mg, Rendement: 90%. F=104°C.
RMN ¹H (CDCl₃, 250 MHz): 1,22 à 1,35 (5H, m), 1,67 (4H, s), 1,76 (3H, s), 4,24 (2H, q, J=7,3Hz), 4,40 (1H, d, J=8,6Hz), 4,52 (1H, d, J=8,6Hz), 6,17 (1H, d, J=16Hz), 6,80 (1H Ar, s), 7,01 (1H Ar, d, J=1,3Hz), 7,08 (1H Ar, s), 7,34 (1H Ar, d, J=1,3Hz), 7,55 (1H , d, J=16Hz).
RMN ¹³C (CDCl₃, 250 MHz): 14,32, 27,31, 31,95, 32,03, 32,09, 32,30, 34,19, 34,76, 35,12, 35,21, 48,35, 60,40, 85,43, 107,23, 117,69, 121,47, 121,49, 127,08, 131,70, 137,32, 137,89, 138,36, 146,10, 153,66, 157,24, 167,22.

### EXEMPLE 13

### ACIDE [5-(3,5,5,8,8-PENTAMETHYL-2,3,5,6,7,8-HEXAHYDRONAPHTO[2,3-B] FURAN-3-YL)-THIOPHENE-3-YL] ACRYLIQUE.

Un mélange de [5-(3,5,5,8,8-pentaméthyl-2,3,5,6,7,8-hexahydronaphto[2,3-b] furan-3-yl)-thiophène-3-yl]-acrylate d'ethyle (430 mg, 1 mmol) et d'hydroxyde de lithium (430mg, mmol) dans le THF (10ml) est chauffé 12h au reflux. Le mélange est acidifié à pH 1 par une solution d'acide chlorhydrique concentré, extrait à l'éther éthylique et lavé à l'eau. Après séchage la phase organique est concentrée à l'évaporateur rotatif sous vide à 40°C, le produit est purifié par chromatographie flash sur colonne de silice.
Solide blanc. Masse: 330mg, Rendement: 82%. F=242°C.
RMN ¹H (CDCl₃, 250 MHz): 1,22 (3H, s), 1,25 (3H, s), 1,27 (3H, s), 1,29 (3H, s), 1,67 (4H, s), 1,77 (3H, s), 4,41 (1H, d, J=8,8Hz), 4,53 (1H, d, J=8,8Hz), 6,17 (1H, d, J=16Hz), 6,80 (1H Ar, s), 7,03 (1H Ar, s), 7,09 (1H Ar, s), 7,40 (1H Ar, s), 7,66 (1H Ar, d, J=16Hz).
RMN ¹³C (CDCl₃, 250 MHz): 27,72, 32,41, 32,48, 32,54, 32,76, 34,65, 35,22, 35,57, 35,65, 48,83, 85,85, 107,72, 117,04, 121,92, 128,67, 132,06, 137,45, 138,41, 141,15, 146,63, 154,44, 157,68, 172,82.

### EXEMPLE 14

### [5-(3,5,5,8,8-PENTAMETHYL-2,3,5,6,7,8-HEXAHYDRONAPHTO[2,3-B] FURAN-3-YL)-THIOPHENE-3-YL]-PROPYNOATE DE METHYL.

### 3-[4-(2,2-Dibromovinyl)-thiophène-2-yl]-3,5,5,8,8-pentaméthyl-2,3,5,6,7,8-hexahydronaphto[2,3-b] furane.

Du tétrabromométhane (375 mg, 1,13 mmol) est additionné à un mélange de triphénylphosphine (440 mg, 1,68 mmol) et de zinc (74 mg, 1,13 mmol) dans le dichlorométhane (5 ml), à 0°C. Le mélange est agité à température ambiante 1h, puis une solution de [5-(3,5,5,8,8-pentaméthyl-2,3,5,6,7,8-hexahydronaphto[2,3-b]furan-3-yl)-thiophen-3-yl]-carbaldéhyde (200 mg, 0,56 mmol) dans le dichlorométhane (2 ml) est additionnée à 0°C. L'agitation est poursuivie 30 mn, puis la suspension est filtrée sur silice. Le filtrat est concentré à l'évaporateur rotatif sous vide.
Solide blanc. Masse: 286mg, Rendement: quantitatif. F=124°C.
RMN ¹H (CDCl₃, 250 MHz): 1,22 (3H, s), 1,25 (3H, s), 1,26 (3H, s), 1,27 (3H, s), 1,66 (4H, s), 1,75 (3H, s), 4,39 (1H, d, J=8,5Hz), 4,51 (1H, d, J=8,5Hz), 6,79 (1H, s), 7,07 (1H, s), 7,10 (1H, s), 7,36 (1H, s), 7,53 (1H, s).
RMN ¹³C (CDCl₃, 250 MHz): 27,84, 32,38, 32,48, 32,75, 34,58, 35,15, 35,52, 35,62, 48,70, 85,87, 88,52, 107,59, 121,89, 124,62, 124,87, 131,97, 136,06, 138,23, 146,45, 152,22, 157,66.

### [5-(3,5,5,8,8-pentaméthyl-2,3,5,6,7,8-hexahydronaphto[2,3-b]furan-3-yl)thiophène-3-yl]-propynoate de méthyl.

Une solution de butyllithium 2,5M dans l'hexane (0,82 ml, 2,45 mmol) est additionnée à une solution de 3-[4-(2,2-dibromovinyl)-thiophène-2-yl]-3,5,5,8,8-pentaméthyl-2,3,5,6,7,8-hexahydronaphto[2,3-b] furane (478 mg, 0,94 mmol) dans le THF (5ml) à -78°C. L'agitation est poursuivie 1h à -78°C puis du chloroformiate de méthyle (80 ul) est additionné. Le mélange est agité à température ambiante 1h, traitée par une solution saturée de chlorure d'ammonium et extrait à l'éther éthylique. Après lavage à l'eau, la phase organique est séchée sur sulfate de magnésium et concentrée à l'évaporateur rotatif sous vide. Le produit est purifié par chromatographie sur colonne.
Solide amorphe. Masse: 90 mg, Rendement: 24%.
RMN ¹H (CDCl₃, 250 MHz): 1,22 (3H, s), 1,24 (3H, s), 1,27 (3H, s), 1,28 (3H, s), 1,66 (4H, s), 1,76 (3H, s), 3,81 (3H, s), 4,38 (1H, d, J=8,5Hz), 4,49 (1H, d, J=8,5Hz), 6,80 (1H, s), 6,93 (1H, d, J=1,1Hz), 7,06 (1H, s), 7,59 (1H, d, J=1,1Hz).
RMN ¹³C (CDCl₃, 250 MHz): 27,00, 31,96, 32,02, 32,12, 32,28, 34,19, 34,77, 35,10, 35,19, 48,23, 52,74, 79,98, 82,30, 85,35, 107,31, 118,33, 121,33, 126,51, 131,43, 132,87, 138,03, 146,30, 153,12, 154,48, 157,23.

### EXEMPLE 15

### ACIDE [5-(3,5,5,8,8-PENTAMETHYL-2,3,5,6,7,8-HEXAHYDRONAPHTO[2,3-B] FURAN-3-YL)-THIOPHENE-3-YL]-PROPYNOIQUE.

Même mode opératoire que pour l'exemple 13 appliqué au [5-(3,5,5,8,8-pentaméthyl-2,3,5,6,7,8-hexahydronaphto[2,3-b]furan-3-yl)-thiophène-3-yl]propynoate de méthyl.
Solide blanc. Rendement: 70%.
RMN ¹H (CDCl₃, 250 MHz): 1,22 (3H, s), 1,25 (3H, s), 1,27 (3H, s), 1,28 (3H, s), 1,66 (4H, s), 1,76 (3H, s), 4,39 (1H, d, J=8,5Hz), 4,50 (1H, d, J=8,5Hz), 6,80 (1H, s), 6,96 (1H, d, 1,3Hz), 7,06 (1H, s), 7,66 (1H, d, J=1,3Hz).
RMN ¹³C (CDCl₃, 250 MHz): 27,34, 32,31, 32,36, 32,47, 32,63, 34,54, 35,13, 35,45, 35,54, 48,59, 80,09, 85,07, 85,68, 107,70, 118,36, 121,68, 126,87, 131,74, 134,14, 138,46, 146,71, 153,68, 157,53, 158,22.

### B. EXEMPLES DE FORMULATIONS

### 1) VOIE ORALE

(a) On prépare la composition suivante sous la forme d'un comprimé de 0,8 g

| | |
|---|---|
| Composé de l'exemple 1 | 0,005 g |
| Amidon prégélatinisé | 0,265 g |
| Cellulose microcristalline | 0,300 g |
| Lactose | 0,200 g |
| Stéarate de magnésium | 0,030 g |

Pour le traitement de l'acné, on administre à un individu adulte 1 à 3 comprimés par jour pendant 3 à 6 mois selon la gravité du cas traité.
(b) On prépare une suspension buvable, destinée à être conditionnée en ampoules de 5 ml

| | |
|---|---|
| Composé de l'exemple 2 | 0,050 g |
| Glycérine | 0,500 g |
| Sorbitol à 70 % | 0,500 g |
| Saccharinate de sodium | 0,010 g |
| Parahydroxybenzoate de méthyle | 0,040 g |
| Arôme q.s. | |
| Eau purifiée q.s.p | 5 ml |

Pour le traitement de l'acné, on administre à un individu adulte 1 ampoule par jour pendant 3 mois selon la gravité du cas traité.
(c) On prépare la formulation suivante destinée à être conditionnée en gélules :

| | |
|---|---|
| Composé de l'exemple 3 | 0,025g |
| Amidon de maïs | 0,060g |
| Lactose q.s.p | 0,300g |

Les gélules utilisées sont constituées de gélatine, d'oxyde de titane et d'un conservateur.
Dans le traitement du psoriasis, on administre à un individu adulte, 1 gélule par jour pendant 30 jours.

### 2) VOIE TOPIQUE

(a) On prépare la crème Eau-dans l'Huile non ionique suivante :

| | |
|---|---|
| Composé de l'exemple 4 | 0,100 g |
| Mélange d'alcools de lanoline émulsifs, de cires et d'huiles raffinés, vendu par la Société BDF sous la dénomination "Eucérine anhydre" | 39,900 g |
| Parahydroxybenzoate de méthyle | 0,075 g |
| Parahydroxybenzoate de propyle | 0,075 g |
| Eau déminéralisée stérile q.s.p | 100,000 g |

Cette crème est appliquée sur une peau psoriatique 1 à 2 fois par jour pendant 30 jours.
(b) On prépare un gel en réalisant la formulation suivante :

| | |
|---|---|
| Composé de l'exemple 5 | 0,050 g |
| Erythromycine base | 4,000 g |
| Butylhydroxytoluène | 0,050 g |
| Hydroxypropylcellulose vendue par la société Hercules sous le nom de "KLUCEL HF" | 2,000 g |
| Ethanol (à 95°) q.s.p | 100,000 g |

Ce gel est appliqué sur une peau atteinte de dermatose ou une peau acnéique 1 à 3 fois par jour pendant 6 à 12 semaines selon la gravité du cas traité.
(c) On prépare une lotion antiséborrhéique en procédant au mélange des ingrédients suivants :

| | |
|---|---|
| Composé de l'exemple 10 | 0,030 g |
| Propylène glycol | 5,000 g |
| Butylhydroxytoluène | 0,100 g |
| Ethanol (à 95°) q.s.p | 100,000 g |

Cette lotion est appliquée deux fois par jour sur un cuir chevelu séborrhéique et on constate une amélioration significative dans un délai compris entre 2 et 6 semaines.
(d) On prépare une composition cosmétique contre les effets néfastes du soleil en procédant au mélange des ingrédients suivants :

| | |
|---|---|
| Composé de l'exemple 11 | 1,000 g |
| Benzylidène camphre | 4,000 g |
| Triglycérides d'acides gras | 31,000 g |
| Monostéarate de glycérol | 6,000 g |
| Acide stéarique | 2,000 g |
| Alcool cétylique | 1,200 g |
| Lanoline | 4,000 g |
| Conservateurs | 0,300 g |
| Propylène glycol | 2,000 g |
| Triéthanolamine | 0,500 g |
| Parfum | 0,400 g |
| Eau déminéralisée q.s.p | 100,000 g |

Cette composition est appliquée quotidiennement, elle permet de lutter contre le vieillissement photoinduit.
(e) On prépare la crème Huile dans l'Eau non ionique suivante :

| | |
|---|---|
| Composé de l'exemple 8 | 0,500 g |
| Vitamine D3 | 0,020 g |
| Alcool cétylique | 4,000 g |
| Monostéarate de glycérol | 2,500 g |
| Stéarate de PEG 50 | 2,500 g |
| Beurre de Karité | 9,200 g |
| Propylène glycol | 2,000 g |
| Parahydroxybenzoate de méthyle | 0,075 g |
| Parahydroxybenzoate de propyle | 0,075 g |
| Eau déminéralisée stérile q.s.p | 100,000 g |

Cette crème est appliquée sur une peau psoriatique 1 à 2 fois par jour pendant 30 Jours.
(f) On prépare un gel topique en procédant au mélange des ingrédients suivants :

| | |
|---|---|
| Composé de l'exemple 15 | 0,050 g |
| Ethanol | 43,000 g |
| α-tocophérol | 0,050 g |
| Polymère carboxyvinylique vendu sous la dénomination "Carbopol 941" par la société "Goodrich" | 0,500 g |
| Triéthanolamine en solution aqueuse à 20 % en poids | 3,800 g |
| Eau | 9,300 g |
| Propylène glycol qsp | 100,000 g |

Ce gel est appliqué dans le traitement de l'acné 1 à 3 fois par jour pendant 6 à 12 semaines selon la gravité du cas traité.
(g) On prépare une lotion capillaire anti-chute et pour la repousse des cheveux en procédant au mélange des ingrédients suivants :

| | |
|---|---|
| Composé de l'exemple 12 | 0,05 g |
| Composé vendu sous la dénomination "Minoxidil" | 1,00 g |
| Propylène glycol | 20,00g |
| Ethanol | 34,92 g |
| Polyéthylèneglycol (masse moléculaire = 400) | 40,00 g |
| Butylhydroxyanisole | 0,01 g |
| Butylhydroxytoluène | 0,02 g |
| Eau qsp | 100,00 g |

On applique cette lotion 2 fois par jour pendant 3 mois sur un cuir chevelu ayant subi une chute de cheveu importante.
(h) On prépare une crème anti-acnéique en procédant au mélange des ingrédients suivants :

| | |
|---|---|
| Composé de l'exemple 9 | 0,050 g |
| Acide rétinoïque | 0,010 g |
| Mélange de stéarates de glycérol et de polyéthylène glycol (75 moles) vendu sous le nom de "Gelot 64" . par la société "GATTEFOSSE" | 15,000 g |
| Huile de noyau polyoxyéthylénée à 6 moles d'oxyde d'éthylène vendue sous le nom de "Labrafil M2130 CS" par la société "GATTEFOSSE" | 8,000 g |
| Perhydrosqualène | 10,000 g |
| Conservateurs | qs |
| Polyéthylèneglycol (masse moléculaire = 400) | 8,000 g |
| Sel disodique de l'acide éthylène-diamine tétracétique | 0,050 g |
| Eau purifiée qsp | 100,000 g |

Cette crème est appliquée sur une peau atteinte de dermatose ou une peau acnéique 1 à 3 fois par jour pendant 6 à 12 semaines.
(i) On prépare une crème huile dans l'eau en réalisant la formulation suivante :

| | |
|---|---|
| Composé de l'exemple 6 | 0,020g |
| 17-valérate de bétamethasone | 0,050 g |
| S-carboxyméthyl cystéine | 3, 000 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de "Tween 20" par la société "ATLAS" | 1,800 g |
| Mélange de mono et distéarate de glycérol vendu sous la dénomination de "Géléol" par la société " GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualène | 18,000 g |
| Mélange de triglycérides caprylique-caprique vendu sous la dénomination de "Miglyol 812" par la société "DYNAMIT NOBEL" | 4,000 g |
| Triéthanolamine (99 % en poids) | 2,500 g |
| Eau q.s.p | 100,000 g |

Cette crème est appliquée 2 fois par jour sur une peau atteinte de dermatose pendant 30 jours.
(j) On prépare la crème de type huile dans l'eau suivante :

| | |
|---|---|
| Acide lactique | 5,000 g |
| Composé de l'exemple 1 | 0,020 g |
| Stéarate de polyoxyéthylène (40 moles d'oxyde d'éthylène) vendu sous le nom de "Myrj 52" par la société "ATLAS" | 4,000 g |
| Monolaurate de sorbitan, polyoxyéthylène à 20 moles d'oxyde d'éthylène vendu sous le nom de Tween 20" par la société "ATLAS" | 1,800 g |
| Mélange de mono et distéarate de glycérol vendu sous la dénomination de "Geleol" par la société "GATTEFOSSE" | 4,200 g |
| Propylène glycol | 10,000 g |
| Butylhydroxyanisole | 0,010 g |
| Butylhydroxytoluène | 0,020 g |
| Alcool cétostéarylique | 6,200 g |
| Conservateurs | q.s. |
| Perhydrosqualène | 18,000 g |
| Mélange de triglycérides caprylique-caprique vendu sous la dénomination de "Miglyol 812" . par la société "DYNAMIT NOBEL" | 4,000 g |
| Eau q.s.p | 100,000 g |

Cette crème est appliquée 1 fois par jour, elle aide à lutter contre le vieillissement qu'il soit photoinduit ou chronologique.

## Revendications

1. Composés biaryles hétérocycliques, caractérisés en ce qu'ils sont représentés par la formule générale (I) suivante : 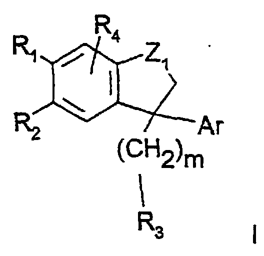 dans laquelle
Z₁ représente O, S, N-r',
R₁ et R₂ pris ensemble forment avec le cycle aromatique adjacent un cycle à 5 ou 6 chainons éventuellement substitué par un ou plusieurs groupes méthyle et/ou éventuellement interrompu par un radical SO, un radical SO₂, un atome d'oxygène ou de soufre,
R₃ représente
(i) un atome d'hydrogène, un radical alkyle ayant de 1 à 6 atomes de carbone, un radical alkényle ayant de 1 à 6 atomes de carbone, un radical alkynyle ayant de 1 à 6 atomes de carbone, un atome d'halogène, un radical cyano ou un radical -O-R₇, R₇ ayant la signification donnée ci-après,
(ii) un radical 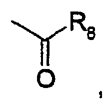 R₈ ayant la signification donnée ci-après,
(iii) un radical 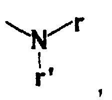 r et r' ayant la signification donnée ci-après,
R4 représente:
(i) un atome d'hydrogène,
(ii) un radical alkyle ayant de 1 à 6 atomes de carbone,
(iii) un atome d'halogène,
(iv) un radical -OR_{7,} R₇ ayant la signification donnée ci-après,
(v) un radical acyle ayant de 1 à 6 atomes de carbone,
Ar représente un radical choisi parmi les radicaux de formules (a)-(h) suivantes: 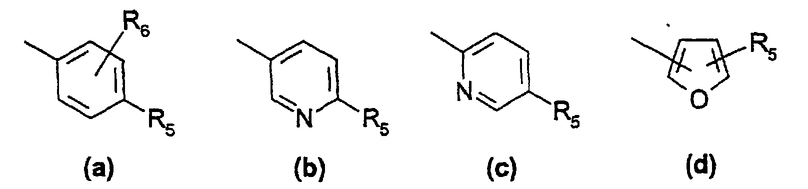 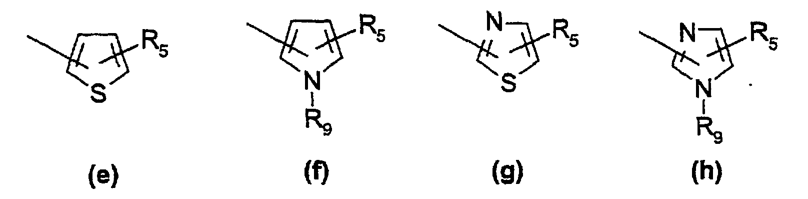 dans lesquelles
R₅ représente :
(i) le radical -CH₃,
(ii) le radical -(CH₂)ₚ-O-R₇,
(iii) un radical 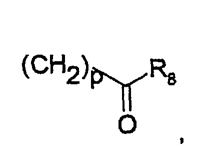
(iv) un radical 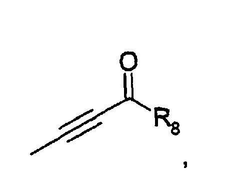
(v) un radical 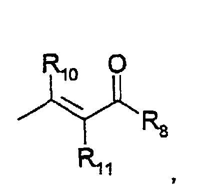
R₇, R₈, R₉, R₁₀, R₁₁ et p ayant les significations données ci-après,
R₆ représente un atome d'hydrogène, un atome d'halogène, un radical alkyle ayant de 1 à 6 atomes de carbone, un radical acyle ayant de 1 à 6 atomes de carbone ou le radical -OR₇, R₇ ayant la signification donnée ci-après,
R₇, identique ou différent, représente un atome d'hydrogène, un radical alkyle ayant de 1 à 6 atomes de carbone, un radical aryle, un radical aralkyle, un radical polyhydroxyalkyle ou un radical polyéther, un radical acyle ayant de 1 à 6 atomes de carbone,
R₈, identique ou différent, représente:
(a) un atome d'hydrogène, un radical alkyle ayant de 1 à 6 atomes de carbone,
(b) un radical 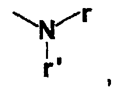 r et r' ayant les significations données ci-après
(c) un radical -OR₁₂
R₉ représente un atome d'hydrogène, un radical alkyle ayant de 1 à 6 atomes de carbone ou un radical acyle ayant de 1 à 6 atomes de carbone,
R₁₀ et R₁₁, identiques ou différents, représentent un atome d'hydrogène ou un radical alkyle ayant de 1 à 6 atomes de carbone
R₁₂ représente un atome d'hydrogène, un radical alkyle ayant de 1 à 6 atomes de carbone, un radical mono ou polyhydroxyalkyle, un radical aryle ou aralkyle éventuellement substitué(s) ou un reste de sucre ou d'aminoacide,
r et r', identiques ou différents, représentent des groupements protecteurs de fonctions amines, un atome d'hydrogène, un radical alkyle ayant de 1 à 6 atomes de carbone, un reste d'amino acide ou de sucre, ou encore pris ensemble, forment un hétérocycle,
m représente 0 ou 1,
p, identique ou différent, représente 0, 1, 2 ou 3,
ainsi que leurs sels, leurs racémiques, leurs isomères optiques purs ou leurs mélanges en toutes proportions.

2. Composés selon la revendication 1, caractérisés en ce que l'une au moins, et de préférence toutes, les conditions ci-dessous sont respectées:
- R₃ est un hydrogène,
- R₄ est un hydrogène,
- R₅ est un radical de formule (iii), (iv) ou (v),
- R₆ est un hydrogène,
- R₈ est un radical OR₁₂,
- R₁₂ est un hydrogène ou un radical alkyle ayant de 1 à 6 atomes de carbone,
- Ar est un radical de formule (a),
- Z₁ est un atome d'oxygène,
- m=1,
- p=0.

3. Composés selon l'une des revendications 1 ou 2, caractérisés en ce qu'ils sont choisis parmi le groupe suivant:
- Acide 4-(3,5,5,8,8-pentaméthyl-2,3,5,6,7,8-hexahydronaphto[2,3-b]furan-3-yl)-benzoïque
- Acide (+) 4-(3,5,5,8,8-pentaméthyl-2,3,5,6,7,8-hexahydronaphto[2,3-b]furan-3-yl)-benzoïque
- Acide (-)-4-(3,5,5,8,8-pentaméthyl-2,3,5,6,7,8-hexahydronaphto[2,3-b]furan-3-yl)-benzoïque
- 4-(3,5,5,8,8-pentaméthyl-2,3,5,6,7,8-hexahydronaphto[2,3-b]furan-3-yl)-benzoate d'éthyle
- (+) 4-(3,5,5,8,8-pentaméthyl-2,3,5,6,7,8-hexahydronaphto[2,3-b]furan-3-yl)-benzoate d'éthyle
- (-) 4-(3,5,5,8,8-pentaméthyl-2,3,5,6,7,8-hexahydronaphto[2,3-b]furan-3-yl)-benzoate d'éthyle
- [5-(3,5,5,8,8-pentamethyl-2,3,5,6,7,8-hexahydronaphto[2,3-b]furan-3-yl)-thiophene-3-yl] carboxylate de méthyle
- acide [5-(3,5,5,8,8-pentamethyl-2,3,5,6,7,8-hexahydronaphto[2,3-b]furan-3-yl)-thiophene-3-yl] carboxylique.
- acide [5-(3,5,5,8,8-pentamethyl-2,3,5,6,7,8-hexahydronaphto[2,3-b]furan-3-yl)-thiophen-2-yl]-carboxylique.
- [5-(3,5,5,8,8-pentamethyl-2,3,5,6,7,8-hexahydronaphto[2,3-b]furan-3-yl)-thiophen-3-yl]-méthanol
- [5-(3,5,5,8,8-pentamethyl-2,3,5,6,7,8-hexahydronaphto[2,3-b]furan-3-yl)-thiophen-3-yl]-carbaldehyde
- [5-(3,5,5,8,8-pentamethyl-2,3,5,6,7,8-hexahydronaphto[2,3-b]furan-3-yl)-thiophene-3-yl]-acrylate d'éthyle.
- acide [5-(3,5,5,8,8-pentamethyl-2,3,5,6,7,8-hexahydronaphto[2,3-b]furan-3-yl)-thiophene-3-yl] acrylique.
- [5-(3,5,5,8,8-pentamethyl-2,3,5,6,7,8-hexahydronaphto[2,3-b]furan-3-yl)-thiophene-3-yl]-propynoate de méthyl.
- acide [5-(3,5,5,8,8-pentamethyl-2,3,5,6,7,8-hexahydronaphto[2,3-b]furan-3-yl)-thiophene-3-yl]-propynoique.
- acide 4-(3,5,5,8,8-pentaméthyl-2,3,5,6,7,8-hexahydronaphto[2,3-b] thiophène-3yl)-benzoïque

4. A titre de médicament, les composés de formule générale (I) selon l'une des revendications 1 à 3.

5. Utlisation d'un composé de formule générale (I) selon l'une des revendications 1 à 3, pour la préparation d'un médicament destiné au traitement des affections respiratoires, dermatologiques, rhumatismales, respiratoires ou ophtalmologiques.

6. Composition pharmaceutique, caractérisée en ce qu'elle comprend un support pharmaceutiquement acceptable et au moins un composé de formule générale (1) selon l'une des revendications 1 à 3.

7. Composition selon la revendication 6, caractérisée en ce qu'elle contient au moins un composé de formule générale (I) à une concentration comprise entre 0,001 et 5 % en poids par rapport au poids total de la composition.

8. Composition cosmique caractérisée en ce qu'elle comprend un support cosmétiquement acceptable et au moins un composé de formule générale (I) selon l'une des revendications 1 à 3.

9. Composition selon la revendication 8, caractérisée en ce qu'elle contient au moins un composés de formule générale (I) à une concentration comprise entre 0,001 et 3 % en poids par rapport au poids total de la composition.

## Patentansprüche

1. Heterocyclische Biarylverbindungen, dadurch gekennzeichnet, daß sie durch die allgemeine Formel I dargestellt werden: 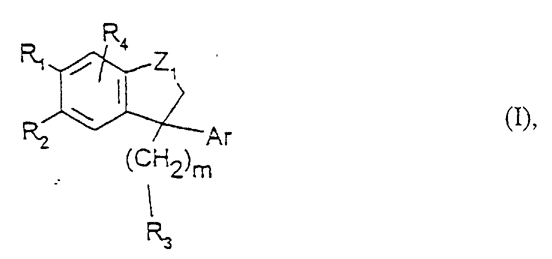 worin:
Z₁ O, S oder N-r' bedeutet,
R₁ und R₂ gemeinsam mit dem angrenzenden aromatischen Ring einen 5- oder 6-gliedrigen Ring bilden, der gegebenenfalls mit einer oder mehreren Methylgruppen substituiert ist und/oder gegebenenfalls von einer Gruppe SO, SO₂, Sauerstoff oder Schwefel unterbrochen ist,
R₃ bedeutet:
(i) Wasserstoff, eine C₁₋₆-Alkylgruppe, eine C₁₋₆-Alkenylgruppe, eine C₁₋₆-Alkinylgruppe, Halogen, Cyano oder eine Gruppe -O-R₇, wobei R₇ die nachstehend angegebene Bedeutung aufweist,
(ii) eine Gruppe 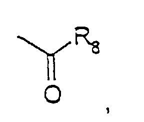 wobei R₈ die nachfolgend angegebene Bedeutung aufweist,
(iii) eine Gruppe 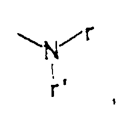 wobei r und r' die nachfolgend angegebenen Bedeutungen aufweisen,
R₄ bedeutet:
(i) Wasserstoff,
(ii) eine C₁₋₆-Alkylgruppe,
(iii) ein Halogenatom,
(iv) eine Gruppe -OR₇, wobei R₇ die nachfolgend angegebenen Bedeutungen aufweist,
(v) eine C₁₋₆-Acylgruppe,
Ar eine Gruppe bedeutet, die unter den Gruppen der folgenden Formeln (a)-(h) ausgewählt ist: 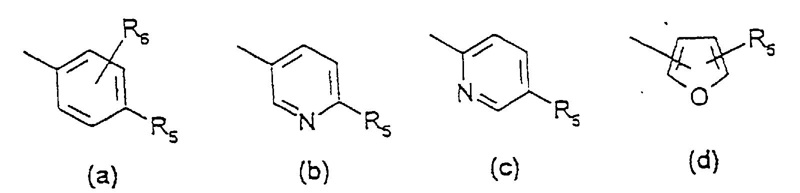 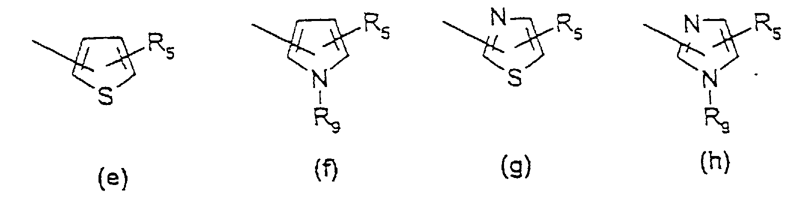 worin bedeuten:
R₅
(i) die Gruppe -CH₃,
(ii) eine Gruppe -(CH₂)ₚ-O-R₇,
(iii) eine Gruppe 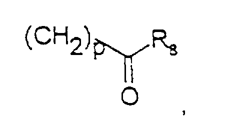
(iv) eine Gruppe 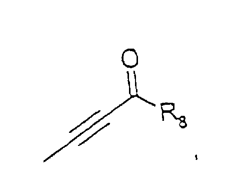
(v) eine Gruppe 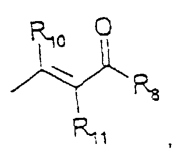
worin die Gruppen R₇, R₈, R₉, R₁₀ und R₁₁ und p die nachfolgend angegebenen Bedeutungen aufweisen,
R₆ Wasserstoff, Halogen, C₁₋₆-Alkyl, C₁₋₆-Acyl oder die Gruppe -OR₇, worin R₇ die nachfolgend angegebenen Bedeutungen aufweist;
R₇, die identisch oder voneinander verschieden sind, Wasserstoff, C₁₋₆-Alkyl, Aryl, Aralkyl, Polyhydroxyalkyl, eine Polyethergruppe oder C₁₋₆-Acyl,
R₈, die identisch oder voneinander verschieden sind:
(a) Wasserstoff oder C₁₋₆-Alkyl,
(b) eine Gruppe 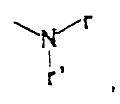 worin r und r' die nachfolgend angegebenen Bedeutungen aufweisen,
(c) eine Gruppe -OR₁₂;
R₉ Wasserstoff, C₁₋₆-Alkyl oder C₁₋₆-Acyl,
R₁₀ und R₁₁, die identisch oder voneinander verschieden sind, Wasserstoff oder C₁₋₆-Alkyl,
R₁₂ Wasserstoff, C₁₋₆-Alkyl, Mono- oder Polyhydroxyalkyl, eine Aryl- oder Aralkylgruppe, die gegebenenfalls substituiert sind, oder einen Zucker- oder Aminosäurerest,
r und r', die identisch oder voneinander verschieden sind, Schutzgruppen für Aminogruppen, Wasserstoff, C₁₋₆-Alkyl, einen Aminosäure- oder Zuckerrest oder sie bilden gemeinsam einen Heterocyclus,
m 0 oder 1,
p, die identisch oder voneinander verschieden sind, 0, 1, 2 oder 3,
sowie deren Salze, Racemate, reinen optischen Isomeren oder deren Gemische in beliebigen Anteilen.

2. Verbindungen nach Anspruch 1, dadurch gekennzeichnet, daß mindestens eine und vorzugsweise alle folgenden Bedingungen erfüllt werden:
- R₃ bedeutet Wasserstoff,
- R₄ bedeutet Wasserstoff,
- R₅ ist eine Gruppe der Formel (iii), (iv) oder (v),
- R₆ ist Wasserstoff,
- R₈ ist eine Gruppe OR₁₂,
- R₁₂ bedeutet Wasserstoff oder C₁₋₆-Alkyl,
- Ar ist eine Gruppe der Formel (a),
- Z₁ bedeutet ein Sauerstoffatom,
- m = 1, und
- p =0.

3. Verbindungen nach einem der Ansprüche 1 oder 2, dadurch gekennzeichnet, daß sie unter den folgenden Verbindungen ausgewählt sind:
- 4-(3,5,5,8,8-Pentamethyl-2,3,5,6,7,8-hexahydronaphtho[2,3-b]furan-3-yl)-benzoesäure,
- (+)-4-(3,5,5,8,8-Pentamethyl-2,3,5,6,7,8-hexahydronaphtho[2,3-b]furan-3-yl)-benzoesäure,
- (-)-4-(3,5,5,8,8-Pentamethyl-2,3,5,6,7,8-hexahydronaphtho[2,3-b]furan-3-yl)-benzoesäure,
- Ethyl-4-(3,5,5,8,8-pentamethyl-2,3,5,6,7,8-hexahydronaphtho[2,3-b]furan-3-yl)-benzoat,
- (+)-Ethyl-4-(3,5,5,8,8-pentamethyl-2,3,5,6,7,8-hexahydronaphtho[2,3-b]furan-3-yl)-benzoat,
- (-)-Ethyl-4-(3,5,5,8,8-pentamethyl-2,3,5,6,7,8-hexahydronaphtho[2,3-b]furan-3-yl)-benzoat,
- [5-(3,5,5,8,8-Pentamethyl-2,3,5,6,7,8-hexahydronaphtho[2,3-b]furan-3-yl)-thiophen-3-yl]-carbonsäuremethylester,
- [5-(3,5,5,8,8-Pentamethyl-2,3,5,6,7,8-hexahydronaphtho[2,3-b]furan-3-yl)-thiophen-3 -yl]-carbonsäure,
- [5-(3,5,5,8,8-Pentamethyl-2,3,5,6,7,8-hexahydronaphtho[2,3-b]furan-3-yl)-thiophen-2-yl]-carbonsäure,
- [5-(3,5,5,8,8-Pentamethyl-2,3,5,6,7,8-hexahydronaphtho[2,3-b]furan-3-yl)-thiophen-3-yl]-methanol,
- [5-(3,5,5,8,8-Pentamethyl-2,3,5,6,7,8-hexahydronaphtho[2,3-b]furan-3-yl)-thiophen-3 -yl]-carbaldehyd,
- 4-(3,5,5,8,8-pentamethyl-2,3,5,6,7,8-hexahydronaphto[2,3-b]thiophen-3-yl)-benzoesäure
- Ethyl-[5-(3,5,5,8,8-pentamethyl-2,3,5,6,7,8-hexahydronaphtho[2,3-b]furan-3-yl)-thiophen-3-yl]-acrylat,
- [5-(3,5,5,8,8-Pentamethyl-2,3,5,6,7,8-hexahydronaphtho[2,3-b]furan-3-yl)-thiophen-3-yl]-acrylsäure,
- Methyl-[5-(3,5,5,8,8-pentamethyl-2,3,5,6,7,8-hexahydronaphtho[2,3-b]furan-3-yl)-thiophen-3-yl]-propinoat,
- [5-(3,5,5,8,8-Pentamethyl-2,3,5,6,7,8-hexahydronaphtho[2,3-b]furan-3-yl)-thiophen-3-yl]-propinsäure.

4. Verbindungen der allgemeinen Formel I nach einem der Ansprüche 1 bis 3 als Arzneimittel.

5. Verwendung einer Verbindung der allgemeinen Formel I nach einem der Ansprüche 1 bis 3 zur Herstellung eines Arzneimittels, das zur Behandlung von respiratorischen, dermatologischen, rheumatischen oder ophthalmologischen Erkrankungen vorgesehen ist.

6. Pharmazeutische Zusammensetzung, dadurch gekennzeichnet, daß sie einen pharmazeutisch akzeptablen Träger und mindestens eine Verbindung der allgemeinen Formel I nach einem der Ansprüche 1 bis 3 enthält.

7. Zusammensetzung nach Anspruch 6, dadurch gekennzeichnet, daß sie mindestens eine Verbindung der allgemeinen Formel I in einer Konzentration im Bereich von 0,001 bis 5 Gew.-%, bezogen auf das Gesamtgewicht der Zusammensetzung enthält.

8. Kosmetische Zusammensetzung, dadurch gekennzeichnet, daß sie einen kosmetisch akzeptablen Träger und mindestens eine Verbindung der allgemeinen Formel I nach einem der Ansprüche 1 bis 3 enthält.

9. Zusammensetzung nach Anspruch 8, dadurch gekennzeichnet, daß sie mindestens eine Verbindung der allgemeinen Formel I bei einer Konzentration im Bereich von 0,001 bis 3 Gew.-%, bezogen auf das Gesamtgewicht der Zusammensetzung, enthält.

## Claims

1. Heterocyclic biaryl compounds, characterized in that they are represented by the following general formula (I): 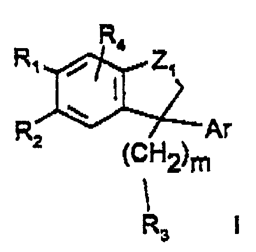 in which
Z₁ represents O, S, N-r',
R₁ and R₂, taken together, form with the adjacent aromatic ring a 5- or 6-membered ring optionally substituted with one or more methyl groups and/or optionally interrupted by an SO radical, an SO₂ radical, an oxygen or sulphur atom,
R₃ represents
(i) a hydrogen atom, an alkyl radical having from 1 to 6 carbon atoms, an alkenyl radical having from 1 to 6 carbon atoms, an alkynyl radical having from 1 to 6 carbon atoms, a halogen atom, a cyano radical or an -O-R₇ radical, with R₇ having the meaning given below,
(ii) a radical 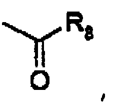 R₈ having the meaning given below,
(iii) a radical 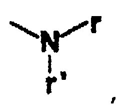 r and r' having the meaning given below,
R₄ represents:
(i) a hydrogen atom,
(ii) an alkyl radical having from 1 to 6 carbon atoms,
(iii) a halogen atom,
(iv) an -OR₇ radical, with R₇ having the meaning given below,
(v) an acyl radical having from 1 to 6 carbon atoms,
Ar represents a radical chosen from the radicals of the following formulae (a)-(h): 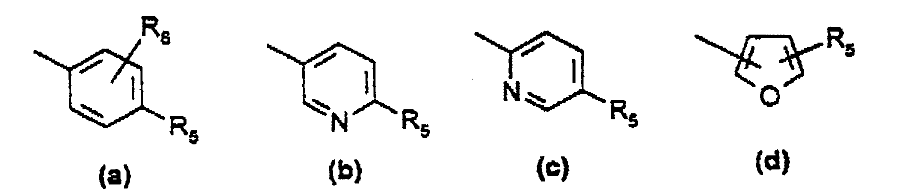 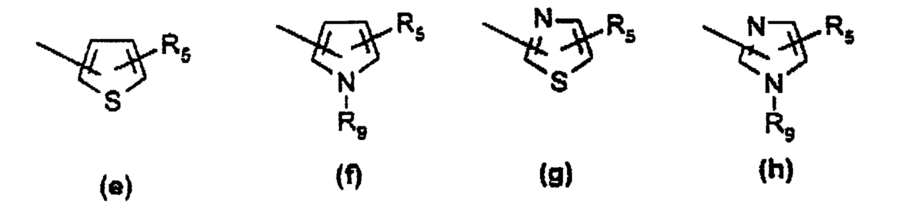 in which
R₅ represents:
(i) the radical -CH₃,
(ii) the radical -(CH₂)ₚ-O-R₇,
(iii) a radical 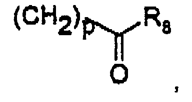
(iv) a radical 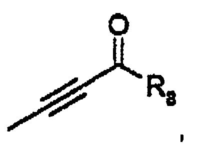
(v) a radical 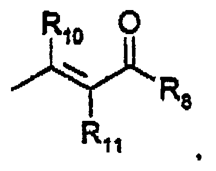
R₇, R₈, R₉, R₁₀, R₁₁ and p having the meanings given below,
R₆ represents a hydrogen atom, a halogen atom, an alkyl radical having from 1 to 6 carbon atoms, an acyl radical having from 1 to 6 carbon atoms or the radical -OR₇, R₇ having the meaning given below,
R₇, identical or different, represents a hydrogen atom, an alkyl radical having from 1 to 6 carbon atoms, an aryl radical, an aralkyl radical, a polyhydroxyalkyl radical or a polyether radical, an acyl radical having from 1 to 6 carbon atoms,
R₈, identical or different, represents:
(a) a hydrogen atom, an alkyl radical having from 1 to 6 carbon atoms,
(b) a radical 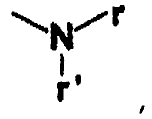 with r and r' having the meanings given below,
(c) a radical -OR₁₂,
R₉ represents a hydrogen atom, an alkyl radical having from 1 to 6 carbon atoms or an acyl radical having from 1 to 6 carbon atoms,
R₁₀ and R₁₁, which are identical or different, represent a hydrogen atom or an alkyl radical having from 1 to 6 carbon atoms,
R₁₂ represents a hydrogen atom, an alkyl radical having from 1 to 6 carbon atoms, a mono- or polyhydroxyalkyl radical, an aryl or aralkyl radical which is (are) optionally substituted, or a sugar or amino acid residue,
r and r', which are identical or different, represent protective groups of amine functions, a hydrogen atom, an alkyl radical having from 1 to 6 carbon atoms, an amino acid or sugar residue or taken together form a heterocycle,
m represents 0 or 1,
p, identical or different, represents 0, 1, 2 or 3,
as well as their salts, their racemates, their pure optical isomers or their mixtures in any proportions.

2. Compounds according to Claim 1, characterized in that at least one, and preferably all, of the conditions below are observed:
- R₃ is a hydrogen,
- R₄ is a hydrogen,
- R₅ is a radical of formula (iii), (iv) or (v),
- R₆ is a hydrogen,
- R₈ is an OR₁₂ radical,
- R₁₂ is a hydrogen or an alkyl radical having from 1 to 6 carbon atoms,
- Ar is a radical of formula (a),
- Z₁ is an oxygen atom,
- m = 1,
- p = 0.

3. Compounds according to either of Claims 1 and 2, characterized in that they are chosen from the following group:
- 4-(3,5, 5, 8,8-pentamethyl-2,3,5,6,7,8-hexahydronaphtho[2,3-b]furan-3-yl)benzoic acid
- (+)-4-(3,5, 5, 8,8-pentamethyl-2,3,5,6,7,8-hexahydronaphtho[2,3-b]furan-3-yl)benzoic acid
- (-)-4-(3,5, 5, 8,8-pentamethyl-2,3,5,6,7,8-hexahydronaphtho[2,3-b]furan-3-yl)benzoic acid
- ethyl 4-(3,5,5,8,8-pentamethyl-2,3,5,6,7,8-hexahydronaphtho[2,3-b]furan-3-yl)benzoate
- 4-(3,5,5,8,8-pentamethyl-2,3,5,6,7,8-hexahydronaphto [2,3-b] thiophen-3yl)-benzoic acid
- ethyl (+)-4-(3,5,5,8,8-pentamethyl-2,3,5,6,7,8-hexahydronaphtho[2,3-b] furan-3-yl)benzoate
- ethyl (-)-4-(3,5, 5,8,8-pentamethyl-2,3,5,6,7,8-hexahydronaphtho[2,3-b]furan-3-yl)benzoate
- methyl [5-(3,5,5,8,8-pentamethyl-2,3,5,6,7,8-hexahydronaphtho[2,3-b]furan-3-yl)thiophen-3-yl]carboxylate
- [5-(3,5,5,8,8-pentamethyl-2,3,5,6,7,8-hexahydronaphtho[2,3-b]furan-3-yl)thiophen-3-yl]carboxylic acid
- [5-(3,5,5,8,8-pentamethyl-2,3,5,6,7,8-hexahydronaphtho [2,3-b]furan-3-yl) thiophen-2-yl]carboxylic acid
- [5-(3,5,5,8,8-pentamethyl-2,3,5,6,7,8-hexahydronaphtho[2,3-b] furan-3-yl) thiophen-3-yl]methanol
- [5-(3,5,5,8,8-pentamethyl-2,3,5,6,7,8-hexahydronaphtho[2,3-b]furan-3-yl)thiophen-3-yl]carbaldehyde
- ethyl [5-(3,5,5,8,8-pentamethyl-2,3,5,6,7,8-hexahydronaphtho[2,3-b]furan-3-yl)thiophen-3-yl]acrylate
- [5-(3,5,5,8,8-pentamethyl-2,3,5,6,7,8-hexahydronaphtho[2,3-b]furan-3-yl)thiophen-3-yl]acrylic acid
- methyl [5-(3,5,5,8,8-pentamethyl-2,3,5,6,7,8-hexahydronaphtho[2,3-b]furan-3-yl)thiophen-3-yl]propynoate
- [5-(3,5,5,8,8-pentamethyl-2,3,5,6,7,8-hexahydronaphtho[2,3-b]furan-3-yl)thiophen-3-yl]propynoic acid.

4. As a medicament, the compounds of general formula (I) according to one of Claims 1 to 3.

5. Use of a compound of general formula (I) according to one of Claims 1 to 3, for the preparation of a medicament for the treatment of respiratory dermatological, rheumatic, respiratory or ophthalmological conditions.

6. Pharmaceutical composition, characterized in that it comprises a pharmaceutically acceptable carrier and at least one compound of general formula (I) according to one of Claims 1 to 3.

7. Composition according to Claim 6, characterized in that it contains at least one compound of general formula (I) at a concentration of between 0.001 and 5% by weight relative to the total weight of the composition.

8. Cosmetic composition characterized in that it comprises a cosmetically acceptable carrier and at least one compound of general formula (I) according to one of Claims 1 to 3.

9. Composition according to Claim 8, characterized in that it contains at least one compound of general formula (I) at a concentration of between O.001 and 3% by weight relative to the total weight of the composition.
